# EUROPÄISCHE PATENTSCHRIFT

(11) **EP 1 214 440 B1**
(45) Veröffentlichungstag und Bekanntmachung des Hinweises auf die Patenterteilung: **28.06.2006**
(21) Anmeldenummer: 00965792.5
(22) Anmeldetag: 29.08.2000
(51) Int. Cl.: C12N 15/90, C12N 15/85, C12N 5/10, A61K 48/00, A61K 31/70, A01K 67/027

(54) **SEQUENZ-SPEZIFISCHE DNA-REKOMBINATION IN EUKARYOTISCHEN ZELLEN**
SEQUENCE-SPECIFIC DNA RECOMBINATION IN EUKARYOTIC CELLS
RECOMBINAISON SEQUENCE-SPECIFIQUE D'ADN DANS DES CELLULES EUCARYOTES

(30) Priorität: 30.08.1999 DE 19941186
(43) Veröffentlichungstag der Anmeldung: 19.06.2002
(60) Teilanmeldung: 06008807.7
(73) Patentinhaber: Dröge, Peter, Singapore 637819 (SG)
(72) Erfinder: DRÖGE, Peter, Singapore 637819 (SG); CHRIST, Nicole, 50677 Köln (DE); LORBACH, Elke, 51103 Köln (DE)
(74) Vertreter: Dehmel, Albrecht
(86) Internationale Anmeldenummer: PCT/DE2000/002947
(87) Internationale Veröffentlichungsnummer: WO 2001/016345

(56) Entgegenhaltungen:
- WO-A-96/40722
- WO-A-97/47758
- DATABASE EMBL SEQUENCES [Online] Accession code LAMCG, 25. Oktober 1995 (1995-10-25) "Bacteriophage lambda genome" XP002164455
- LORBACH E. ET AL.: "Site-specific recombination in human cells catalyzed by phage lambda integrase mutants." J. MOL. BIOL., Bd. 296, 10. März 2000 (2000-03-10), Seiten 1175-1181, XP002164453
- CHRIST N. & DRÖGE P.: "Alterations in the directionality of lambda site-specific recombination catalyzed by mutant integrases in vivo." J. MOL. BIOL., Bd. 288, 21. Mai 1999 (1999-05-21), Seiten 825-836, XP002164454

### SEQUENZPROTOKOLL

<110> Dröge Dr., Peter
<120> Sequenz-spezifische DNA-Rekombination in eukaryotischen Zellen
<130> DRO-001 PCT
<140> xx
   <141> 2000-08-29
<150> DE 199 41 186.7
   <151> 1999-08-30
<160> 5
<170> Patent In Ver. 2.1
<210> 1
   <211> 21
   <212> DNA
   <213> Escherichia coli
<400> 1
   ctgctttttt atactaactt g 21
<210> 2
   <211> 243
   <212> DNA
   <213> Bacteriophage lambda
<400> 2
   tctgttacag gtcactaata ccatctaagt agttgattca tagtgactgc atatgttgtg 60
   ttttacagta ttatgtagtc tgttttttat gcaaaatcta atttaatata ttgatattta 120
   tatcatttta cgtttctcgt tcagcttttt tatactaagt tggcattata aaaaagcatt 180
   gcttatcaat ttgttgcaac gaacaggtca ctatcagtca aaataaaatc attatttgat 240
   ttc                                                               243
<210> 3
   <211> 102
   <212> DNA
   <213> Escherichia coli
<400> 3
   ctgctttttt atactaagtt ggcattataa aaaagcattg cttatcaatt tgttgcaacg 60
   aacaggtcac tatcagtcaa aataaaatca ttatttgatt tc                    102
<210> 4
   <211> 162
   <212> DNA
   <213> Escherichia coli
<400> 4
   tctgttacag gtcactaata ccatctaagt agttgattca tagtgactgc atatgttgtg 60
   ttttacagta ttatgtagtc tgttttttat gcaaaatcta atttaatata ttgatattta 120
   tatcatttta cgtttctcgt tcagcttttt tatactaact tg                    162
<210> 5
   <211> 243
   <212> DNA
   <213> Künstliche Sequenz
<220>
   <223> Beschreibung der künstlichen Sequenz:Oligonukleotid
<400> 5
   tctgttacag gtcactaata ccatctaagt agttgattca tagtgactgc atatgttgtg 60
   ttttacagta ttatgtagtc tgttttttat gcaaaatcta atttaatata ttgatattta 120
   tatcatttta cgtttctcgt tcagcttttt gatactaagt tggcattata aaaaagcatt 180
   gcttatcaat ttgttgcaac gaacaggtca ctatcagtca aaataaaatc attatttgat 240
   ttc                                                               243

## Beschreibung

Die vorliegende Erfindung betrifft ein Verfahren zur Sequenz-spezifischen Rekombination von DNA in eukaryotischen Zellen in vitro umfassend das Einführen in eine Zelle einer ersten DNA-Sequenz, wie sie in Anspruch 1 definiert ist, das Einführen in eine Zelle einer zweiten DNA-Sequenz, wie sie in Anspruch 1 definiert und das Durchführen der Sequenz-spezifischen Rekombination durch Einwirken einer Bakteriophagen *Lambda*-Integrase Int. Eine bevorzugte Ausführungsform betrifft ein Verfahren, ferner umfassend das Durchführen einer zweiten Sequenz-spezifischen Rekombination von DNA durch Einwirken einer Int und eines Xis-Faktors. Die vorliegende Erfindung betrifft weiterhin Vektoren und ihre Verwendung als Arzneimittel.

Die kontrollierte Manipulation eukaryotischer Genome ist eine wichtige Methode zur Erforschung der Funktion(en) bestimmter Gene im lebenden Organismus. Darüberhinaus spielt sie eine Rolle bei gentherapeutischen Verfahren im medizinischen Bereich. Die Herstellung von transgenen Tierstämmen, die Veränderung von Genen oder Genabschnitten (sog. "gene targetting") und die zielgerichtete Integration fremder DNA in das Genom höherer Eukaryoten sind in diesem Zusammenhang von besonderer Bedeutung. Durch die Charakterisierung und Anwendung von Sequenz-spezifischen Rekombinationssystemen konnten diese Technologien in letzter Zeit verbessert werden.

Konservative, Sequenz-spezifische DNA-Rekombinasen werden in zwei Familien eingeteilt. Mitglieder der ersten, der sog. "Integrase"-Familie katalysieren die Trennung und Neuverknüpfung von DNA-Strängen zwischen zwei definierten Nukleotidsequenzen, die im folgenden als Rekombinationssequenzen bezeichnet werden. Diese können entweder auf zwei verschiedenen oder auf einem DNA-Molekül liegen. Es kommt dann zur inter- bzw. intramolekularen Rekombination. Im letzten Fall hängt das Resultat der Reaktion von der jeweiligen Anordnung der Rekombinationssequenzen zueinander ab. Befinden sich die Rekombinationssequenzen in invertierter, d.h. entgegengesetzter, Orientierung zueinander, kommt es zur Inversion des dazwischen liegenden DNA-Segments. Befinden sich die Rekombinationssequenzen als direkte, d.h. gleichgerichtete, Wiederholungen auf einem DNA-Substrat, kommt es zur Deletion. Bei der intermolekularen Rekombination, d.h. wenn die beiden Rekombinationssequenzen auf zwei verschiedenen DNA-Molekülen plaziert sind, kann es zu einer Fusion der zwei DNA-Moleküle kommen. Während Mitglieder der Integrase-Familie üblicherweise sowohl intra- als auch intermolekulare Rekombination katalysieren, sind Rekombinasen der zweiten Familie, der sog. "Invertasen/Resolvasen", nur zur intramolekularen Rekombination fähig.

Die Rekombinasen, die zur Zeit hauptsächlich zur Manipulation eukaryotischer Genome benutzt werden, gehören zur Integrase-Familie. Es sind dies die Cre-Rekombinase des Bakteriophagen *P1* und die Fip-Rekombinase aus Hefe; vgl. Müller, U. (1999) Mech. Develop., **82,** pp. 3. Die Rekombinationssequenzen, an die Cre-Rekombinase bindet, werden als *loxP* bezeichnet. *loxP* ist eine 34 bp lange Nukleotidsequenz, die aus je zwei 13 bp langen invertierten Nukleotidsequenzen und einem dazwischen liegenden 8 bp langen Spacer besteht; vgl. Hoess, R. et al. (1985) J. Mol. Biol., **181**, pp. 351. Die Bindungssequenzen für Flp, als *FRT* bezeichnet, sind ähnlich aufgebaut, unterscheiden sich jedoch von *loxP*; vgl. Kilby, J. et al. (1993) Trends Genet., **9**, pp. 413. Die Rekombinationssequenzen können daher nicht gegeneinander ausgetauscht werden, d.h. Cre kann keine *FRT-* und FLP keine *loxP*-Sequenzen rekombinieren. Beide Rekombinationssysteme sind über weite Distanzen aktiv, d.h. das zu invertierende oder deletierende DNA-Segment, flankiert von zwei *loxP-* oder *FRT*-Sequenzen, kann mehrere 10.000 Basenpaare (kb) lang sein.

Mit diesen beiden Systemen wurde z.B. gewebespezifische Rekombination im Mausmodell, chromosomale Translokation in Pflanzen und Tieren, und eine kontrollierte Induktion der Genexpression erzielt; vgl. Übersichtsartikel von Müller, U. (1999) Mech. Develop., **82**, pp. 3. So wurde das Gen der DNA-Polymerase β in bestimmten Geweben der Maus deletiert; vgl. Gu, H. et al. (1994) Science, **265**, pp. 103. Ein weiteres Beispiel ist die spezifische Aktivierung des Onkogens vom DNA-Tumorvirus SV40 in den Augenlinsen der Maus, was zur Tumorbildung in ausschließlich diesen Geweben führte. Die Cre-*loxP* Strategie wurde darüberhinaus auch im Zusammenhang mit induzierbaren Promotoren verwendet. Hierdurch wurde z.B. die Expression der Rekombinase mit einem Interferon-induzierbaren Promotor reguliert, was zur Deletion eines bestimmten Gens in der Leber und nicht - oder in nur geringem Ausmaß - in anderen Geweben führte; vgl. Kühn, R. et al. (1995) Science, **269,** pp. 1427.

Zwei Mitglieder der Invertase/Resolvase-Famille sind bislang zur Manipulation eukaryotischer Genome verwendet worden. Eine Mutante der Invertase Gin vom Bakteriophagen *Mu* kann ohne Kofaktoren die Inversion eines DNA-Fragments in Pflanzenprotoplasten katalysieren. Allerdings wurde festgestellt, daß diese Mutante hyperrekombinativ ist, d.h. auch an anderen als ihren natürlichen Rekombinationssequenzen DNA-Strangtrennungen katalysiert. Dies führt zu ungewollten, teilweise lethalen Rekombinationsereignissen im Genom der Pflanzenprotoplasten. Die β-Rekombinase aus *Streptococcus pyogenes* katalysiert Rekombination zwischen zwei direkt wiederholten Rekombinationssequenzen in Zellkulturen der Maus, was zum Ausschneiden des Segments führt. Allerdings wurde neben der Deletion auch Inversion nachgewiesen, womit der kontrollierte Einsatz dieses Systems zur Manipulation eukaryotischer Genome untauglich wird.

Auch die Manipulation eukaryotischer Genome mit der Cre- bzw. Flp-Rekombinase weist erhebliche Nachteile auf. Bei der Deletion, d.h. der Rekombination von zwei direkt wiederholt vorliegenden *loxP-* oder *FRT*-Rekombinationssequenzen in einem Genom, kommt es zum irreversiblen Verlust des dazwischen liegenden DNA-Segments. Befindet sich auf diesem DNA-Segment ein Gen, geht dieses folglich für die Zelle und den Organismus für immer verloren. Die Wiederherstellung des Ursprungszustandes für eine erneute Analyse der Genfunktion in diesen Zellen, zum Beispiel in einem späteren Entwicklungsstadium des Organismus, ist deshalb unmöglich. Der unwiderrufliche Verlust des DNA-Segments durch Deletion kann durch die Inversion des betreffenden DNA-Segments umgangen werden. Hierdurch könnte ein Gen inaktiviert werden, ohne daß es verloren ginge, und zu einem späteren Zeitpunkt in der Entwicklung oder im adulten Tier erneut durch eine zeitlich regulierbare Expression der Rekombinase via Rückrekombination angeschaltet werden. Der Nachteil bei der Anwendung der Cre- bzw. Flp-Rekombinase bei diesem modifizierten Verfahren liegt jedoch darin, daß die Inversion nicht reguliert werden kann, da die Rekombinationssequenzen nicht durch den Rekombinationsvorgang verändert werden. Es kommt folglich zu wiederholten Rekombinationsvorgängen, wodurch die Inaktivierung des betreffenden Gens durch Inversion des betreffenden DNA-Segments in nur einigen, max. 50%, der Zielzellen herbeigeführt wird. Man hat versucht, dieses Problem zumindest teilweise zu lösen, indem mutierte *loxP*-Sequenzen konstruiert wurden, die nach einmaliger Rekombination für weitere Reaktionen nicht mehr verwendet werden können. Der Nachteil liegt hierbei jedoch in der Einmaligkeit der Reaktion, d.h. nach Inaktivierung eines Gens durch Inversion kann eine nachträgliche Aktivierung durch die Rückrekombination nicht mehr erfolgen.

Ein weiterer Nachteil der Flp-Rekombinase ist deren reduzierte Hitzestabilität bei 37°C, was die Effizienz der Rekombinationsreaktion in höheren Eukaryoten, z.B. der Maus mit einer Körpertemperatur von ca. 39°C, erheblich einschränkt. Hier wurden Flp-Mutanten konstruiert, die eine höhere Hitzestabilität als die der Wildtyp-Rekombinase aufweisen, jedoch immer noch eine geringere Rekombinationseffizienz verglichen mit der Cre-Rekombinase zeigen.

Eine Verwendung von Sequenz-spezifischen Rekombinasen liegt ferner im medizinischen Bereich z.B. in der Gentherapie, bei der die Rekombinasen ein gewünschtes DNA-Segment stabil und gezielt in das Genom der jeweiligen menschlichen Zielzelle integrieren müssen.
Sowohl Cre als auch Flp können intermolekulare Rekombination katalysieren. Beide Rekombinasen rekombinieren eine Plasmid-DNA, die eine Kopie ihrer jeweiligen Rekombinationssequenz trägt, mit einer zuvor stabil über homologe Rekombination inserierten entsprechenden Rekombinationssequenz im eukaryotischen Genom. Wünschenswert ist jedoch, daß diese Reaktion mit einer "natürlich" vorkommenden Rekombinationssequenz im eukaryotischen Genom durchführbar ist. Da *loxP* und *FRT* 34 bzw. 54 Nukleotide lang sind, ist ein Vorkommen dieser Rekombinationssequenzen als Bestandteil des Genoms aus statistischen Gründen extrem unwahrscheinlich. Selbst bei Vorhandensein ergibt sich der Nachteil der zuvor beschriebenen Rückreaktion, d.h. nach erfolgreicher Integration kann sowohl die Cre- als auch die Flp-Rekombinase das inserierte DNA-Segment durch intramolekulare Rekombination wieder ausschneiden.

Somit besteht eine Aufgabe der vorliegenden Erfindung darin, ein einfaches und regulierbares Rekombinationssystem und die benötigten Arbeitsmittel zur Verfügung zu stellen. Ferner besteht eine Aufgabe der vorliegenden Erfindung darin, ein Rekombinationssystem und die benötigten Arbeitsmittel zur Verfügung zu stellen, das eine stabile und gezielte Integrierung einer ausgewählten DNA-Sequenz durchführen kann.

Die Aufgaben werden durch den in den Patentansprüchen definierten Gegenstand gelöst.

Das erfindungsgemäße Verfahren wird durch die folgenden Figuren erläutert.

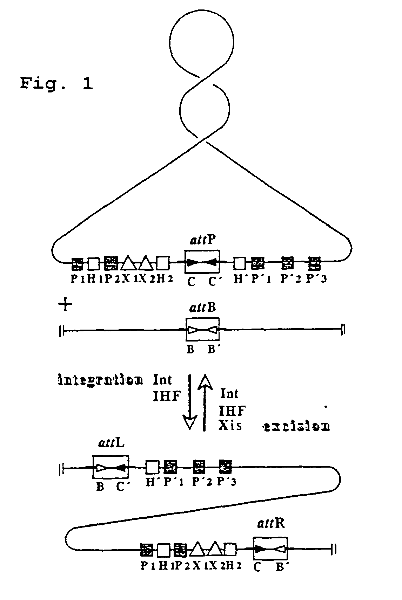

Figur 1 zeigt eine schematische Darstellung der durch die Integrase Int katalysierten Rekombinationsreaktionen, Integration und Excision. Dargestellt ist eine superhelikal gespannte Plasmid DNA (oben), die eine Kopie der Rekombinationssequenz *att*P trägt. Diese enthält fünf sog. Arm-Bindungsstellen für Int (P1, P2, P1', P2', P3'), zwei "core"-Int-Bindungsstellen (C und C'; markiert durch schwarze Pfeile), drei Bindungsstellen für IHF (H1, H2, H'), zwei Bindungsstellen für Xis (X1, X2) und die sog. "overlap"-Region (offenes Rechteck), wo der eigentliche DNA-Strangausstausch stattfindet. Die Partnersequenz für *att*P, *att*B, ist darunter auf einem linearen DNA-Segment dargestellt. Sie besteht aus zwei "core"-Bindungsstellen für Int (B und B'; markiert durch offene Pfeile) und der "overlap"-Region. Die Rekombination zwischen *att*B und *att*P erfordert Int und IHF. Sie führt zur Integration des Plasmids in das *att*B-tragende DNA-Segment. Hierdurch entstehen zwei neue, hybride Rekombinationssequenzen, *att*L und *att*R, die als Zielsequenzen für die Excision dienen. Diese Reaktion benötigt neben Int und IHF einen weiteren Kofaktor, Xis, der vom *lambda* Phagen kodiert wird.

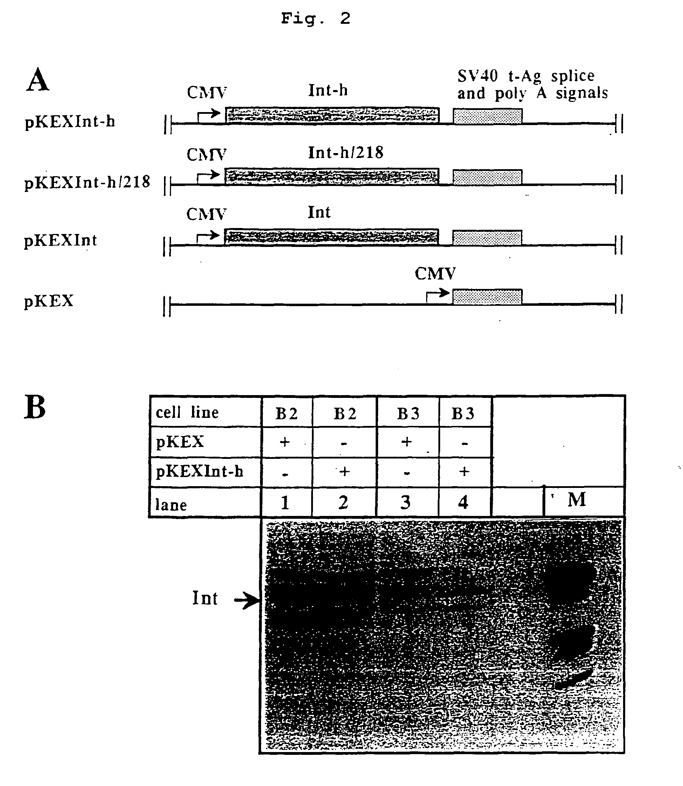

Figur 2 A zeigt eine schematische Darstellung der Integrase Expressionsvektoren und Figur 2 B eine schematische Darstellung einer Western-Analyse. (A): Der Vektor pKEXInt enthält das Wildtyp-Integrase-Gen, der Vektor pKEXInt-h enthält das Gen der Mutante Int-h, und der Vektor pKEXInt-h/218 enthält das Gen der Mutante Int-h/218. Der Kontrollvektor (pKEX) enthält kein Int-Gen. Die jeweiligen Gene für Wildtyp-Integrase (Int) und der beiden Mutanten (Int-h und Int-h/218) sind als graue Balken dargestellt. Hinter den kodierenden Regionen befinden sich Signalsequenzen zur RNA-Prozessierung, die eine erhöhte intrazelluläre Stabilität der jeweiligen m-RNA garantieren sollen (gepunktete Rechtecke) und als SV40, t-Ag splice and poly A signals bezeichnet sind. Die Expression der Integrase-Gene erfolgt durch den menschlichen Cytomegalo Virus (CMV)-Promoter. (B): Nach Einbringen des jeweiligen Vektors, wie angezeigt, in die Reporterzelllinien B2 und B3 wurden Zelllysate präpariert und Proteine in einer SDS-Polyacrylamid-Gelelektrophorese nach ihrem Molekulargewicht aufgetrennt. Die Anwesenheit des Int-h Proteins wurde durch polyklonale Mausantikörper, die gegen Wildtyp-Int gerichtet sind, sichtbar gemacht (Spuren 2 und 4). Die Position von Int im Gel ist durch einen Pfeil markiert.

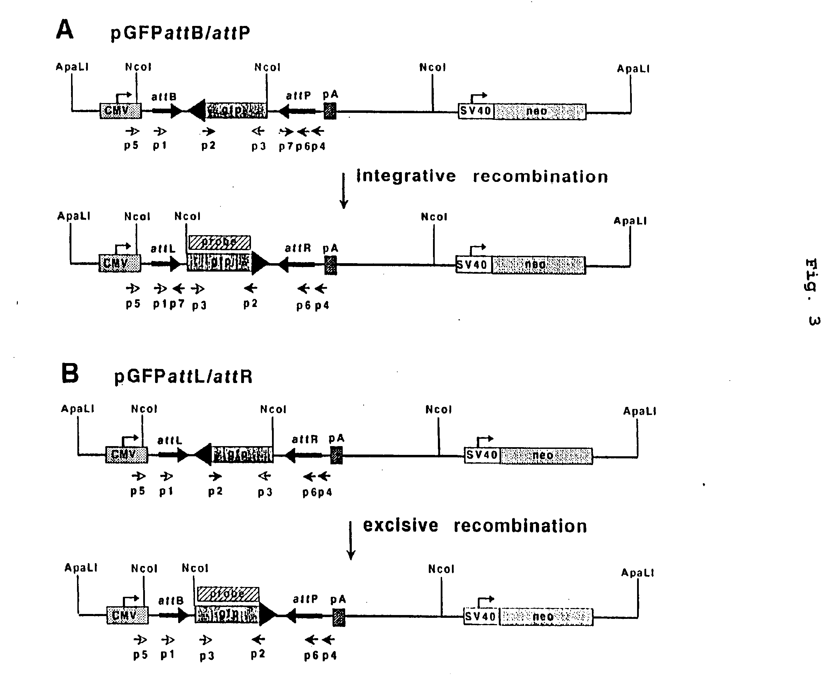

Figur 3 zeigt eine schematische Darstellung der Substratvektoren. (A): pGFP*att*B/*att*P. Dargestellt ist der durch ApaLI linearisierte Vektor. Die großen, schwarzen Pfeile markieren die Position und Orientierung der beiden Rekombinationssequenzen, *att*B und *att*P. Sie flankieren das GFP(green fluorescent protein)-Gen, das in invertierter Orientierung zum CMV-Promoter vorliegt. pA bezeichnet das polyA-Signal. Zusätzlich befindet sich auf dem Vektor das "neo"-Resistenzgen, welches durch den frühen SV40-Promoter exprimiert wird und die Selektion stabiler Reporterzellinien ermöglicht. Restriktionsschnittstellen für das Enzym NcoI sind ebenfalls gekennzeichnet. Die integrative Rekombination zwischen *att*B und *att*P führt zur Inversion des GFP-Gens und damit zu dessen Expression. Die kleinen, offenen und geschlossenen Pfeile markieren die Position und Orientierung der einzelnen PCR-Primer und sind als p1 bis p7 bezeichnet. (B): pGFP*att*L/*att*R. Der Vektor ist im Aufbau identisch mit pGFP*att*B/*att*P, enthält jedoch *att*L und *att*R anstelle von *att*B und *att*P. Das GFP-Gen liegt zum CMV-Promotor in 3'-5'-Orientierung vor. Der schraffierte Kasten bezeichnet die Position der Sonde (probe), die für die Southem-Analyse verwendet wurde.

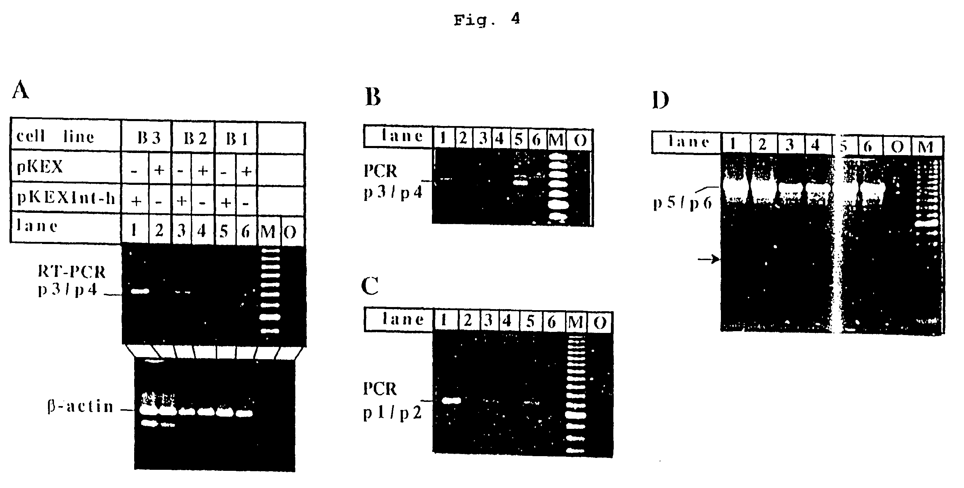

Figur 4 A bis D zeigt in einer schematischen Darstellung den Nachweis der integrativen Rekombination in Reporterzellinien durch PCR nach Auftrennung der DNA-Moleküle in Agarose-Gelen (1,2% w/v), in denen DNA durch Anfärben mit Ethidiumbromid sichtbar gemacht wurde. (A): Reverse Transkriptase PCR (RT-PCR). Die Vektoren pKEX und pKEXInt-h (siehe Figur 2A) wurden separat in die jeweilige Reporterzelllinie B1 bis B3 durch Elektroporation eingebracht. Die RT-PCR-Analyse, ausgehend von isolierter polyA m-RNA, zeigt das erwartete Produkt durch Primerpaar p3/p4 (siehe Figur 3) nur, wenn die Zellen mit pKEXInt-h behandelt wurden (Spur 1, 3 und 5). Zur Kontrolle des RNA-Gehalts wurde das β-Aktin-Gen aus denselben RNA-Präparationen amplifiziert. Spur M: DNA-Längenstandard; Spur O: Kontroll RT-PCR ohne RNA-Template. (B,C): Genomische PCR-Analyse. Genomische DNA wurde aus den jeweiligen Zellinien 72 Stunden nach Elektroporation isoliert und mit Primerpaaren p3/p4 (siehe Figur 3) und p1/p2 (siehe Figur 3) amplifiziert. Die Numerierung und Bezeichnung der Spuren entspricht der in (Fig. 4 A). (D): Test auf Deletion. Isolierte genomische DNA wurde mit Primerpaar p5/p6 (siehe Figur 3) amplifiziert. Die Position des durch Deletion, anstelle von Inversion, erwarteten PCR Produkts (420 bp) ist mit einem Pfeil markiert. Die Numerierung und Bezeichnung der Spuren ist wie in Fig. 4 A.

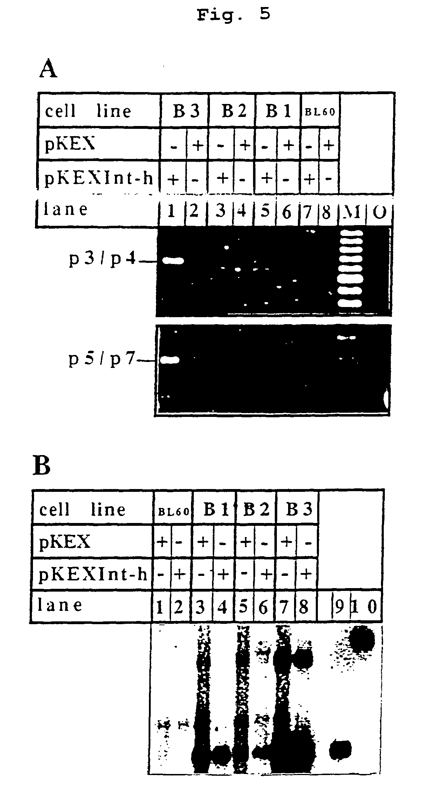

Figur 5 A und B zeigt in einer schematischen Darstellung den Nachweis der Inversion in Reporterzellinien durch PCR und Southern Hybridisierung nach Auftrennung der DNA-Moleküle in Agarose-Gelen (1,2% w/v). (A): PCR-Analyse. Eine Fraktion genomischer DNA, die aus den mit den Vektoren pKEX und pKEXInt-h behandelten Zellinien B1, B2, B3 und BL60 isoliert wurde, wurde mit Primerpaaren p3/p4 und p5/p7 (siehe Figur 3) amplifiziert. Die auf die Integrase-katalysierte Inversion des GFP-Gens zurückzuführenden PCR-Produkte, sind in den Spuren 1, 3 und 5 zu sehen. Spur M: DNA Längenstandard; Spur O: Kontroll-PCR ohne genomische DNA. (B) Southern Analyse: Die Restfraktion der in Fig. 5 A analysierten DNA wurde mit dem Restriktionsenzym NcoI inkubiert, durch eine Agarose-Gelelektrophorese nach Molekulargewicht aufgetrennt und anschließend auf eine Nitrozellulose-Membran übertragen. Zum Nachweis der Rekombination wurden GFP-tragende DNA-Fragmente durch eine radioaktiv markierte Sonde (siehe Figur 3 B) sichtbar gemacht. Spur 9: unrekombiniertes pGFP*att*B/*att*P; Spur 10: rekombiniertes pGFP*att*B/*att*P.

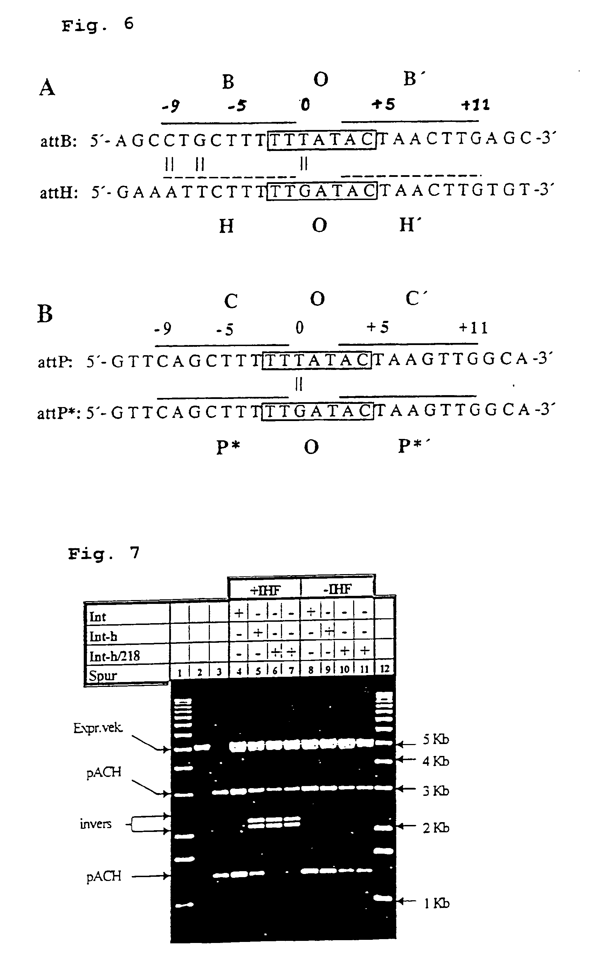

Figur 6 A zeigt eine Darstellung von Nukleinsäuresequenzen die *att*B bzw. *att*H umfassen. Figur 6 B zeigt eine Darstellung von Teil-Sequenzen von *att*P und *att*P*. (A): Sequenzvergleich zwischen *att*B und *att*H. Die Int "core"-Bindungsstellen B und B' in *att*B sind mit einem Strich über den Sequenzen markiert. Die Int "core"-Bindungstellen H und H' in *att*H sind mit einer gestrichelten Linie über den Sequenzen markiert. Die "overlap"-Sequenzen sind durch offene Rechtecke gekennzeichnet. Sequenzunterschiede sind mit senkrechten Doppelstrichen markiert. Die Numerierung der Reste in den Core- und Overlap-Regionen bezieht sich auf das von Landy und Ross ((1977), Science, **197,** pp. 1147) definierte Zentrum des Overlaps, das mit O bezeichnet ist. *Att*B bzw. *att*H ist die Sequenz von -9 bis +11. (B): Sequenzvergleich zwischen Teil-Sequenzen von *att*P und *att*P*, die den *att*B bzw. *att*H-Sequenzen entsprechen. Die Bezeichnungen sind wie in Fig. 6 A gewählt.

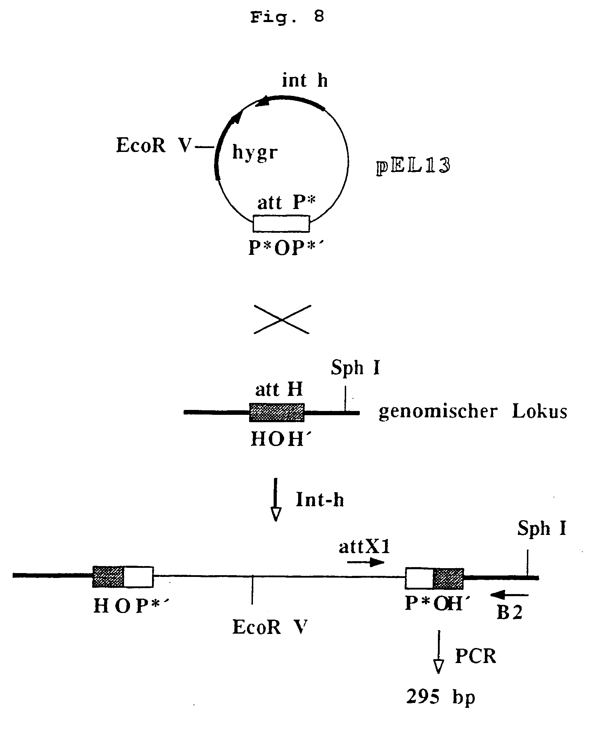

Figur 7 zeigt in einer schematischen Darstellung den Nachweis der Rekombination zwischen *att*H und *att*P* auf dem Vektor pACH in *E. coli* nach Auftrennung in einer Agarose-Gelelektrophorese. Der Substratvektor pACH wurde zusammen mit den jeweiligen prokaryotischen Expressionsvektoren für Int, Int-h oder Int-h/218 in *E. coli* Stamm CSH26 oder CSH26 delta IHF co-transformiert. Plasmid-DNA wurde 36 Stunden nach Selektion isoliert, mit den Restriktionsenzymen HindIII und AvaI inkubiert, durch Agarose-Gelelektrophorese aufgetrennt und sichtbar gemacht. Die Position der durch Inversion enstandenen Restriktionsfragmente ist mit "invers" gekennzeichnet. Die Position der nicht rekombinierten DNA ist mit pACH markiert. Spuren 1 und 12: DNA Längenstandards; Spuren 2 und 3: Expressionsvektor bzw. unrekombinierte pACH-DNA; Spuren 4 bis 7: DNA isoliert aus CSH26; Spuren 8 bis 11: DNA isoliert aus CSH26 delta IHF.

Figur 8 zeigt eine schematische Darstellung der Strategie zur Integration des Vektors pEL13 in den genomischen *att*H Lokus und das Prinzip der Nachweismethode. Der Integrationsvektor pEL13 trägt ein Resistenzgen (Pfeil gekennzeichnet mit "hygr"), das Gen für Int-h (Pfeil gekennzeichnet mit "int h") unter der Kontrolle des CMV-Promoters und eine Kopie von *att*P* (offenes Rechteck gekennzeichnet mit "att P*/P*OP*'"). Nach Einbringen des Vektors in BL60 Zellen durch Elektroporation wird Int-h exprimiert (siehe Figur 2 B). Die Rekombinase katalysiert dann die intermolekulare Rekombination zwischen *att*P* und chromosomal lokalisiertem *att*H (schraffiertes Rechteck gekennzeichnet mit "att H/HOH'"). Dies führt zur Integration des Vektors pEL13 in das Genom der BL60-Zellen. Die Zellen, die den Vektor stabil aufgenommen haben, können selektioniert und durch PCR mit dem Primerpaar *att*X1/B2 (Pfeile gekennzeichnet mit "*att*X1" und "B2") identifiziert werden. EcoR V und SpH I bezeichnen Restriktionsschnittstellen für die entsprechenden Restriktionsenzyme.

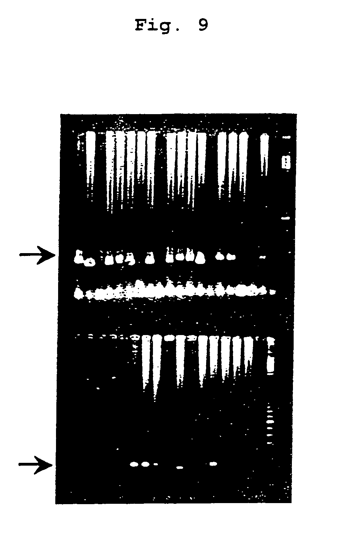

Figur 9 zeigt in einer schematischen Darstellung den Nachweis der intermolekularen Rekombination zwischen *att*P* (pEL13) und *att*H in BL60-Zellen. Genomische DNA wurde nach Elektroporation von pEL13 und anschließender mehrwöchiger Selektion aus 31 verschiedenen Zellpopulationen isoliert und mit Primerpaar attX1/B2 (siehe Fig. 8) amplifiziert. Die PCR-Produkte wurden durch Agarose-Gelelektrophorese aufgetrennt und sichtbar gemacht. Die Position des erwarteten Produkts (295 bp) in den Gelen ist durch den Pfeil markiert. Die Produkte wurden anschließend durch DNA-Sequenzierung weiter analysiert. Am rechten Rand der Gele befindet sich ein Längenstandard.

Der hier verwendete Ausdruck "Transformation" oder "transformieren" bezeichnet jegliches Einführen einer Nukleinsäuresequenz in eine Zelle. Das Einführen kann z.B. eine Transfektion oder Lipofektion sein, oder durch das Calcium-Verfahren, Elektroschock-Verfahren oder eine Oocyteninjektion durchgeführt werden. Der Ausdruck "Transformation" oder "transformieren" bedeutet hier auch das Einführen einer viralen Nukleinsäuresequenz, umfassend z.B. die Rekombinationssequenz(en) und ein therapeutisches Gen oder Genfragment, auf dem für den jeweiligen Virus natürlicherweise durchgeführten Weg. Die virale Nukleinsäuresequenz muß dabei nicht als nackte Nukleinsäuresequenz vorliegen sondern kann in einer viralen Proteinhülle verpackt sein. Der Ausdruck bezeichnet daher nicht nur das Verfahren, das üblicherweise unter dem Begriff Transformation oder transformieren verstanden wird.

Der hier verwendete Ausdruck "Derivat" bezeichnet *att*B- und *att*P-Sequenzen und *att*L- und *att*R-Sequenzen, die gegenüber den natürlicherweise vorkommenden Rekombinationssequenzen Modifikationen in Form von einer oder mehreren, maximal sechs, insbesondere zwei, drei, vier oder fünf Substitutionen aufweisen.

Der hier verwendete Ausdruck "Homologe" oder "homolog" oder "ähnlich" in Bezug auf Rekombinationssequenzen bezeichnet eine Nukleinsäuresequenz, die mit den natürlicherweise vorkommenden Rekombinationssequenzen zu etwa 70%, vorzugsweise zu etwa 80%, besonders bevorzugt zu etwa 85%, ferner besonders bevorzugt zu etwa 90%, ferner besonders bevorzugt zu etwa 95%, und insbesondere bevorzugt zu 99% identisch ist.

Der hier verwendete Ausdruck "Vektor" bezeichnet natürlich vorkommende oder künstlich erschaffene Konstrukte zur Aufnahme, Vermehrung, Expression oder Übertragung von Nukleinsäuren, z.B. Plasmide, Phagemide, Cosmide, künstliche Chromosomen, Bakteriophagen, Viren oder Retroviren.

Die Integrase (üblicherweise und im folgenden "Int" bezeichnet) des Bakteriophagen *Lambda* gehört, wie Cre und Flp, zur Integrase-Familie der konservativen Sequenz-spezifischen DNA-Rekombinasen. Int katalysiert die integrative Rekombination zwischen zwei unterschiedlichen Rekombinationssequenzen, *att*B und *att*P. *Att*B umfaßt 21 Nukleotide und ist ursprünglich aus dem *E. coli* Genom isoliert worden; vgl. Mizuuchi, M. und Mizuuchi, K. (1980) Proc. Natl. Acad. Sci. USA, **77**, pp. 3220. *Att*P ist dagegen mit 243 Nukleotiden erheblich länger und kommt natürlicherweise im Genom des Bakteriophagen *Lambda* vor; vgl. Landy, A. und Ross, W. (1977) Science, **197**, pp. 1147. Die Int-Rekombinase hat insgesamt sieben Bindungstellen in *att*P und zwei in *att*B. Die biologische Funktion von Int ist die Sequenz-spezifische Integration des zirkulären Phagengenoms in den Lokus *att*B auf dem *E. coli* Chromosom. Für die integrative Rekombination benötigt Int einen Protein-Kofaktor, den sog. "Integration Host Factor" (üblicherweise und im folgenden "IHF" bezeichnet); vgl. Kikuchi, Y. und Nash, H. (1978) J.

Biol. Chem., **253,** 7149. IHF wird für den Aufbau eines funktionellen Rekombinationskomplexes mit *att*P benötigt. Ein zweiter Kofaktor für die Integrationsreaktion ist negatives DNA-Supercoiling von *att*P. Die Rekombination zwischen *att*B und *att*P führt schließlich zur Bildung zweier neuer Rekombinationssequenzen, *att*L und *att*R, die als Substrat und Erkennungssequenz für eine weitere Rekombinationsreaktion, die Excisionsreaktion, dienen. Eine umfassende Übersicht der Bakteriophagen *Lambda*-Integration ist z.B. in Landy, A. (1989) Annu. Rev. Biochem., **58,** pp. 913, zu finden.

Die excisive Rekombination (Excision) des Phagengenoms aus dem Bakteriengenom wird ebenfalls von der Int-Rekombinase katalysiert. Hierzu ist neben Int und IHF unbedingt ein weiterer Kofaktor nötig, der ebenfalls vom Bakteriophagen *Lambda* kodiert wird. Es handelt sich um die Excisionase (üblicherweise und im folgenden "Xis" bezeichnet), die zwei Bindungsstellen in *att*R hat; vgl. Gottesman, M. und Weisberg, R. (1971) The Bacteriophage Lambda, Cold Spring Harbor Laboratory, pp.113. Für die excisive Rekombination ist im Gegensatz zur integrativen Rekombination negatives DNA-Supercoiling der Rekombinationssequenzen nicht erforderlich, steigert jedoch die Effizienz der Rekombinationsreaktion. Eine weitere Verbesserung der Effizienz der Excisionsreaktion kann durch einen zweiten Ko-Faktor, FIS (Factor for Inversion Stimulation) erzielt werden, der in Zusammhang mit Xis wirken kann; vgl. Landy, A. (1989) Annu. Rev. Biochem., 58, pp.913. Die Excision ist genetisch die exakte Umkehrreaktion der Integration, d.h. es werden wiederum *att*B und *att*P generiert. Eine umfassende Übersicht der Bakteriophagen *Lambda*-Excision ist z.B. in Landy, A. (1989) Annu. Rev. Biochem., **58**, pp. 913, zu finden.

Ein Aspekt der vorliegenden Erfindung betrifft ein Verfahren zur Sequenz-spezifischen Rekombination von DNA in eukaryotischen Zellen, umfassend a) das Einführen in eine Zelle einer ersten DNA-Sequenz, wie sie in Anspruch 1 definiert ist, b) das Einführen in eine Zelle einer zweiten DNA-Sequenz, wie sie in Anspruch 1 definiert ist und c) das Durchführen der Sequenz-spezifischen Rekombination durch Einwirken einer Bakteriophagen *Lambda*-Integrase Int. Bevorzugt ist ein Verfahren, wobei die erste DNA-Sequenz eine *att*B-Sequenz gemäß SEQ ID NO:1 oder deren Derivat umfaßt, und die zweite DNA-Sequenz eine *att*P-Sequenz gemäß SEQ ID NO:2 oder deren Derivat umfaßt. Weiter bevorzugt ist ein Verfahren, wobei die erste DNA-Sequenz eine *att*L-Sequenz gemäß SEQ ID NO:3 oder deren Derivat umfaßt, und die zweite DNA-Sequenz eine *att*R-Sequenz gemäß SEQ ID NO:4 oder deren Derivat umfaßt, wobei in Schritt c) die Sequenz-spezifische Rekombination durch Einwirken einer Int und eines XIS-Faktors durchgeführt wird.

Das erfindungsgemäße Verfahren kann dabei nicht nur mit der natürlicherweise vorkommenden *att*B- und/oder *att*P-Sequenz oder *att*L- und/oder *att*R-Sequenz sondern auch mit modifizierten, z.B. substituierten, *att*B- und/oder *att*P-Sequenzen oder *att*L- und/oder *att*R-Sequenzen durchgeführt werden. So wurde z.B. integrative Rekombination des Bakteriophagen *Lambda* und *E. coli* zwischen *att*P- und *att*B-homologen Sequenzen (Mutanten der Wildtyp-Sequenzen) beobachtet, die eine oder eine Kombination folgender Substitutionen an folgenden Positionen in *att*B aufweisen: G, T (an Position -9); A, C, G (-8); C, A, T (-7); T, G, A (-6); C, A (-5); A (-4); G, A (-3); A, C, G (-2); A, C, G (-1); A, C, G (0); T, C, G (+1); A, C, G (+2); T, G, C (+3); A, G, T (+4); A, C, G (+5); G, T (+6); G, T (+7); G, T, A (+8); C, G, A (+9); C, G, A (+10); T, A, C (+11) (Nash, H. (1981) Annu. Rev. Genet., 15, pp. 143; Nussinov, R. und Weisberg, R. (1986) J. Biomol. Struct. Dynamics, **3**, pp 1134) und/oder in *att*P aufweisen: T (an Position +1); C (+2) und A (+4); vgl. Nash, H. (1981) Annu. Rev. Genet., **15**, pp.143.

Die vorliegende Erfindung betrifft somit ein Verfahren, in dem die verwendete *att*B-Sequenz gegenüber der natürlicherweise vorkommenden *att*B-Sequenz gemäß SEQ ID NO:1 und die verwendete *att*P-Sequenz gegenüber der natürlicherweise vorkommenden *att*P-Sequenz gemäß SEQ ID NO:2 eine oder mehrere Substitutionen aufweisen. Ferner betrifft die vorliegende Erfindung ein Verfahren, in dem die verwendete *att*L-Sequenz gegenüber der natürlicherweise vorkommenden *att*L-Sequenz gemäß SEQ ID NO:3 und die verwendete *att*R-Sequenz gegenüber der natürlicherweise vorkommenden *att*R-Sequenz gemäß SEQ ID NO:3 eine oder mehrere Substitutionen aufweisen. Bevorzugt ist ein Verfahren, wobei die Rekombinationssequenzen eine, zwei, drei, vier oder fünf Substitutionen aufweisen. Die Substitutionen können sowohl in der Overlap-Region (siehe Fig. 6 A, offenes Rechteck) als auch in der Core-Region (siehe Fig. 6 A, Strich) vorkommen. Auch kann die gesamte Overlap-Region, die sieben Nukleotide umfaßt, ausgetauscht werden. Besonders bevorzugt ist ein Verfahren, bei dem in der *att*B- und *att*P-Sequenz entweder in der Core-Region oder in der Overlap-Region Substitutionen eingeführt werden. Bevorzugt ist die Einführung von einer Substitution in der Overlap-Region und die gleichzeitige Einführung von einer oder zwei Substitutionen in der Core-Region.

Für das erfindungsgemäße Verfahren ist es nicht erforderlich, daß bei einer Substitution in *att*B die entsprechende Substitution in *att*P, oder bei einer Substitution in *att*L die entsprechende Substitution in *att*R, eingeführt wird oder umgekehrt. Eine Modifikation durch Substitution in Rekombinationssequenzen ist so zu wählen, daß die Rekombination trotz der Modifikation(en) durchführbar ist. Beispiele für derartige Substitutionen sind z.B. in den Veröffentlichungen von Nash, H. (1981), *supra* und Nussinov, R. und Weisberg, R. (1986), *supra* aufgeführt und sind nicht als abschließend zu betrachten. Weitere Modifikationen können leicht z.B. über Mutagenese-Verfahren eingeführt und durch Testrekombinationen auf ihre Anwendung überprüft werden.

Die vorliegende Erfindung betrifft somit ferner ein Verfahren, in dem entweder die verwendete *att*B-Sequenz gegenüber der natürlicherweise vorkommenden *att*B-Sequenz gemäß SEQ ID NO:1 oder die verwendete *att*P-Sequenz gegenüber der natürlicherweise vorkommenden *att*P-Sequenz gemäß SEQ ID NO:2, oder entweder die verwendete *att*L-Sequenz gegenüber der natürlicherweise vorkommenden *att*L-Sequenz gemäß SEQ ID NO:3 oder die verwendete *att*R-Sequenz gegenüber der natürlicherweise vorkommenden *att*R-Sequenz gemäß SEQ ID NO:4, eine oder mehrere Substitutionen aufweisen. Eine oder mehrere Substitutionen in einer der Rekombinationssequenzen muß somit nicht die entsprechende Substitution in der anderen Rekombinationssequenz zur Folge haben.

In einer bevorzugten Ausführungsform des erfindungsgemäßen Verfahrens umfaßt die *att*B-Sequenz 21 Nukleotide und stimmt mit der ursprünglich aus dem *E. coli* Genom isolierten Sequenz überein (Mizuuchi, M. und Mizuuchi, K. (1980) Proc. Natl. Acad. Sci. USA, **77**, pp. 3220) und umfaßt die *att*P-Sequenz 243 Nukleotide und stimmt mit der ursprünglich aus dem Genom des Bakteriophagen *Lambda* isolierten Sequenz überein; vgl. Landy, A. und Ross, W. (1977) Science, **197**, pp. 1147.

In einer weiteren bevorzugten Ausführungsform des erfindungsgemäßen Verfahrens umfaßt die *att*L-Sequenz 102 Nukleotide und die *att*R-Sequenz 162 Nukleotide und stimmen beide mit den ursprünglich aus dem *E. coli* Genom isolierten Sequenzen überein; vgl. Landy, A. (1989) Annu. Rev. Biochem., 58, pp.913.

Zur Durchführung des erfindungsgemäßen Verfahrens kann die erste DNA-Sequenz neben der Rekombinationssequenz weitere DNA-Sequenzen umfassen, die eine Integration in einen gewünschten Zielort im Genom der eukaryotischen Zelle erlauben. Diese Integration verläuft über die homologe Rekombination, die durch zellinteme Rekombinationsmechanismen vermittelt wird. Dazu müssen die weiteren DNA-Sequenzen zur Zielort-DNA homolog sein und sowohl 3' als auch 5' von der *att*B bzw. *att*L-Sequenz liegen. Wie hoch der Grad der Homologie und wie lang die jeweiligen 3'- und 5'-Sequenzen sein müssen, damit die homologe Rekombination mit einer hinreichenden Wahrscheinlichkeit abläuft, ist dem Fachmann bekannt; vgl. Übersichtsartikel von Capecchi, M. (1989) Science, **244**, pp. 1288.

Auch die zweite DNA-Sequenz mit der *att*P bzw. *att*R-Rekombinationssequenz kann DNA-Sequenzen umfassen, die für eine Integration in einen gewünschten Zielort mittels homologer Rekombination erforderlich sind. Für das erfindungsgemäße Verfahren kann sowohl die erste und/oder die zweite DNA-Sequenz die weiteren DNA-Sequenzen umfassen. Bevorzugt ist ein Verfahren, bei dem beide DNA-Sequenzen die weiteren DNA-Sequenzen umfassen.

Das Einführen der ersten und zweiten DNA-Sequenz, mit oder ohne weitere DNA-Sequenzen, kann sowohl nacheinander als auch in einer Cotransformation durchgeführt werden, wobei die DNA-Sequenzen auf zwei verschiedenen DNA-Molekülen vorliegen. Bevorzugt ist ein Verfahren, bei dem die erste und die zweite DNA-Sequenz, mit oder ohne weitere DNA-Sequenzen, auf einem einzigen DNA-Molekül vorhanden sind und in die eukaryotischen Zellen eingeführt werden. Ferner kann die erste DNA-Sequenz in eine Zelle und die zweite DNA-Sequenz in eine andere Zelle eingeführt werden, wobei die Zellen anschließend verschmolzen werden. Unter Verschmelzen ist das Kreuzen von Organismen und Zellfusion im weiteren Sinn zu verstehen.

Das erfindungsgemäße Verfahren kann z.B. verwendet werden, um bei einer intramolekularen Rekombination das zwischen den indirekt orientierten Rekombinationssequenzen liegende DNA-Segment zu invertieren. Ferner kann das erfindungsgemäße Verfahren verwendet werden, um bei einer intramolekularen Rekombination das zwischen den direkt orientierten Rekombinationssequenzen liegende DNA-Segment zu deletieren. Die Rekombinationssequenzen liegen direkt orientiert vor, wenn die Sequenzen jeweils in 5'-3'- oder in 3'-5'-Richtung eingebaut werden. Die Rekombinationssequenzen liegen indirekt orientiert vor, wenn z.B. die *att*B-Sequenz in 5'-3'-Richtung und die *att*P-Sequenz in 3'-5'-Richtung eingebaut wird. Werden die Rekombinationssequenzen über homologe Rekombination jeweils in eine Intronsequenz 5' und 3' eines Exons placiert und die Rekombination durch das Einwirken der Integrase durchgeführt, so wird das Exon bei indirekt orientierten Rekombinationssequenzen umgedreht (invertiert) bzw. bei direkt orientierten Rekombinationssequenzen deletiert. Dadurch kann z.B. das vom entsprechenden Gen codierte Polypeptid seine Aktivität oder Funktion verlieren oder die Transkription durch die Inversion oder Deletion gestoppt werden, so daß kein (vollständiges) Transkript entsteht. Auf diese Weise kann z.B. die biologische Funktion des kodierten Polypeptids untersucht werden.

Die erste und/oder zweite DNA-Sequenz kann/können jedoch noch weitere Nukleinsäuresequenzen umfassen, die ein oder mehrere Polypeptid(e) von Interesse kodieren. So kann z.B. mit den Rekombinationssequenzen ein Strukturprotein, ein Enzym oder ein regulatorisches Protein in das Genom eingeführt werden, das nach erfolgter intramolekularer Rekombination transient exprimiert wird. Das eingeführte Polypeptid kann endogen oder exogen sein. Ferner kann ein Markerprotein eingeführt werden. Dem Fachmann ist bewußt, daß diese Auflistung von Anwendungen des erfindungsgemäßen Verfahrens nur beispielhaft und nicht abschließend ist. Beispiele für erfindungsgemäße Anwendungen, die mit den bisher verwendeten Cre- und Flp-Rekombinasen durchgeführt wurden, sind z.B. in dem Übersichtsartikel von Kilby, N. et al., (1993), Trends Genet., **9**, pp.413, zu finden.

Das erfindungsgemäße Verfahren kann weiterhin verwendet werden, um bei der intramolekularen Rekombination auf episomalen Substraten DNA-Segmente auf Vektoren zu deletieren oder zu invertieren. Eine Deletionsreaktion könnte z.B. dazu verwendet werden, Verpackungssequenzen aus sogenannten Helferviren zu entfernen. Diese Methode hat ein breites Anwendungsgebiet bei der industriellen Produktion viraler Vektoren für gentheraupeutische Ansätze; vgl. Hardy, S. et al., (1997), J. Virol., 71, pp.1842.

Die intermolekulare Rekombination führt zur Fusion zweier DNA-Moleküle, die jeweils eine Kopie von *att*B und *att*P oder *att*L und *att*R besitzen. So kann z.B. *att*B zunächst über homologe Rekombination in einen bekannten, gut charakterisierten genomischen Lokus einer Zelle eingeführt werden. In diese genomische *att*B-Sequenz kann anschließend ein *att*P-tragender Vektor über intermolekulare Rekombination integriert werden. Bevorzugt ist bei diesem Verfahren die Expression der Integrase-Mutante Int-h/218, dessen Gen sich auf einem zweiten DNA-Vektor, der ko-transfiziert wird, befindet. Auf dem *att*P-tragenden Vektor können sich weitere Sequenzen befinden, z.B. ein Gen für ein bestimmtes Markerprotein, flankiert von *loxP-*/*FRT*-Sequenzen. Mit diesem Ansatz kann z.B. erreicht werden, daß bei vergleichenden Expressionsanalysen verschiedener Gene in einem Zelltyp diese nicht durch positive oder negative Einflüsse des jeweiligen genomischen Integrationsortes beeinflußt werden.

Für die Durchführung des erfindungsgemäßen Verfahrens muß eine Integrase auf die Rekombinationssequenzen einwirken. Dabei kann die Integrase oder das Integrase-Gen und/oder der Xis-Faktor oder das Xis-Faktor-Gen bereits vor Einführen der ersten und zweiten DNA-Sequenz in der eukaryotischen Zelle vorliegen, zwischen der Einführung der ersten und zweiten DNA-Sequenz oder nach Einführung der ersten und zweiten DNA-Sequenz eingeführt werden. Die für die Sequenz-spezifische Rekombination verwendete Integrase wird bevorzugt in der Zelle exprimiert, in der sie die Reaktion durchführt. Dazu wird eine dritte DNA-Sequenz, umfassend ein Integrase-Gen, in die Zellen eingeführt. Wird die Sequenz-spezifische Rekombination mit *att*L/*att*R durchgeführt, kann zusätzlich noch ein Xis-Faktor-Gen (vierte DNA-Sequenz) in die Zellen eingeführt werden. Insbesondere bevorzugt ist ein Verfahren, in dem die dritte und/oder vierte DNA-Sequenz ins eukaryotische Genom der Zelle über homologe Rekombination oder wahllos integriert wird. Ferner bevorzugt ist ein Verfahren, in dem die dritte und/oder vierte DNA-Sequenz regulatorische DNA-Sequenzen umfaßt, die eine räumliche und/oder zeitliche Expression des Integrase-Gens und/oder Xis-Faktor-Gens bewirken.

Räumliche Expression bedeutet in diesem Fall, daß die Rekombinase bzw. der Xis-Faktor nur in einem bestimmten Zelltyp, z.B. Leberzellen, Nierenzellen, Nervenzellen oder Zellen des Immunsystems, durch die Verwendung von zelltypspezifischen Promotoren exprimiert wird und nur in diesen Zellen die Rekombination katalysiert. Eine zeitliche Expression bei der Regulation der Integrase/Xis-Faktor-Expression kann durch Promotoren erzielt werden, die ab oder in einem bestimmten Entwicklungsstadium oder zu einem bestimmten Zeitpunkt im adulten Organismus aktiv sind. Ferner kann die zeitliche Expression durch die Verwendung von induzierbaren Promotoren, z.B. durch Interferon- oder Tetrazyklin-abhängige Promotoren, erzielt werden; vgl. Übersichtsartikel von Müller, U. (1999) Mech. Develop.,**82**, pp. 3.

Die beim erfindungsgemäßen Verfahren verwendete Integrase kann sowohl die Wildtyp-Integrase als auch eine modifizierte Integrase des Bakteriophagen *Lambda* sein. Da die Wildtyp-Integrase die Rekombinationsreaktion nur mit Hilfe eines Kofaktors, nämlich IHF durchführen kann, ist für das erfindungsgemäße Verfahren die Verwendung einer modifizierten Integrase bevorzugt. Wird die Wildtyp-Integrase für das erfindungsgemäße Verfahren verwendet, wird für die Rekombinationsreaktion zusätzlich IHF benötigt. Die modifizierte Integrase ist derart modifiziert, daß sie die Rekombinationsreaktion ohne IHF durchführen kann. Die Herstellung von modifizierten Polypeptiden und die Durchmusterung auf die gewünschte Aktivität sind Stand der Technik und einfach durchzuführen; vgl. Erlich, H. (1989) PCR Technology. Stockton Press. Bevorzugte Bakteriophagen *Lambda*-Integrasen sind zwei Int-Mutanten, die als Int-h und Int-h/218 bezeichnet werden; vgl. Miller et al. (1980) Cell, **20**, pp. 721; Christ, N. und Dröge, P. (1999) J. Mol. Biol., **288**, pp. 825. Int-h enthält einen Lysin-Rest anstelle des Glutamat-Restes an Position 174 gegenüber Wildtyp-Int. Int-h/218 enthält einen weiteren Lysin-Rest anstelle eines Glutamat-Restes an Position 218 und wurde über PCR-Mutagenese des Int-h Gens hergestellt. Diese Mutanten können sowohl Rekombination zwischen *att*B/*att*P als auch zwischen *att*L/*att*R ohne die Kofaktoren IHF, Xis und negativem Supercoiling in *E. coli* und *in vitro,* d.h. mit gereinigten Substraten im Reagenzglas, katalysieren. In eukaryotischen Zellen benötigen die Mutanten für die Rekombination zwischen *att*L/*att*R allein den Kofaktor Xis. Eine weitere Verbesserung der Effizienz der Rekombination zwischen *att*L/*att*R kann durch einen weiteren Ko-Faktor, z.B. FIS erzielt werden. Die Mutante Int-h/218 ist bevorzugt, da sie die kofaktorunabhängige integrative Reaktion mit erhöhter Effizienz katalysieren kann; vgl. Christ, N. und Dröge, P. (1999) J. Mol. Biol., **288,** pp. 825.

Das erfindungsgemäße Verfahren kann in allen eukaryotischen Zellen durchgeführt werden. Die Zellen können z.B. in einer Zellkultur vorliegen und alle Arten von pflanzlichen oder tierischen Zellen umfassen. Z.B. können die Zellen nicht menschliche Oocyten, nicht menschliche embryonale Stammzellen, hämatopoietische Stammzellen oder jede Art von differenzierten Zellen sein. Bevorzugt ist ein Verfahren, in dem die eukaryotische Zelle eine Säugerzelle ist. Besonders bevorzugt ist ein Verfahren, in dem die Säugerzelle eine menschliche Zelle (mit Ausnahme von Keimzellen), Affen-, Maus-, Ratten-, Kaninchen-, Hamster-, Ziege-, Kuh-, Schaf- oder Schweinezelle ist.

Eine bevorzugte Ausführungsform der vorliegenden Erfindung betrifft ferner ein Verfahren, in dem gegebenenfalls eine zweite Sequenz-spezifische Rekombination von DNA durch Einwirken einer Bakteriophagen *Lambda*-Integrase und eines Xis-Faktors durchgeführt wird. Die zweite Rekombination benötigt die durch eine erste Rekombination von *att*B und *att*P oder deren Derivaten generierten *att*L- und *att*R-Sequenzen. Daher ist die zweite Sequenz-spezifische Rekombination beschränkt auf ein Verfahren, das in der ersten Sequenz-spezifischen Rekombination *att*B- und *att*P-Sequenzen oder deren Derivaten verwendet. Sowohl die Wildtyp-Integrase als auch die Int-Mutanten können ohne Zugabe weiterer Faktoren nur die sogenannte integrative Rekombination katalysieren, d.h. sie rekombinieren *att*B mit *att*P und nicht *att*L mit *att*R, wenn diese stabil im Genom der Zellen integriert sind. Die Wildtyp-Integrase benötigt dabei für die sogenannte Excisions-Rekombination die Faktoren IHF, Xis und negatives Supercoiling. Die Int-Mutanten Int-h und Int-h/218 benötigen für die Excisions-Rekombination nur den Faktor Xis. Somit ist es möglich, zwei Rekombinationsreaktionen kontrolliert nacheinander ablaufen zu lassen, wenn weitere Faktoren für die zweite Rekombinationsreaktion, nämlich die Excisions-Reaktion, in den Zellen vorhanden sind. Im Zusammenspiel mit anderen, bereits verwendeten Rekombinationssystemen, können hier neue Strategien zur kontrollierten Manipulation höherer Eukaryotengenome erarbeitet werden. Dies ist möglich, da die verschiedenen Rekombinationssysteme ausschließlich ihre eigenen Rekombinationssequenzen benutzen.

So kann z.B. das Int-System benutzt werden, um *lox*P- und/oder *FRT*-Sequenzen gezielt in einen genomischen Lokus eines Eukaryotengenoms zu integrieren und anschließend durch kontrollierte Expression von Cre und/oder Flp ein Gen zu aktivieren bzw. inaktivieren. Ferner kann das Int-System benutzt werden, um *loxP*-/*FRT*-Sequenzen nach Gebrauch, d.h. Rekombination durch die entsprechende Rekombinase, aus einem Genom zu entfernen.

Bevorzugt ist ein Verfahren, in dem ferner eine weitere DNA-Sequenz, umfassend ein Xis-Faktor-Gen, in die Zellen eingeführt wird. Besonders bevorzugt ist ein Verfahren, in dem die weitere DNA-Sequenz ferner eine regulatorische DNA-Sequenz umfaßt, die eine räumliche und/oder zeitliche Expression des Xis-Faktor-Gens bewirkt.

So kann z.B. nach erfolgreicher integrativer, intramolekularer Rekombination (Inversion) durch Int, was zur Aktivierung/Inaktivierung eines Gens in einem bestimmten Zelltyp geführt hat, dieses Gen zu einem späteren Zeitpunkt durch die induzierbare räumliche und/oder zeitliche Expression von Xis bei gleichzeitiger Expression von Int wieder inaktiviert oder aktiviert werden.

Die Erfindung betrifft ferner die Verwendung einer *att*B-Sequenz gemäß SEQ ID NO:1 oder deren Derivat und einer *att*P-Sequenz gemäß SEQ ID NO:2 oder deren Derivat, oder einer *att*L-Sequenz gemäß SEQ ID NO:3 oder deren Derivat und einer *att*R-Sequenz gemäß SEQ ID NO:4 oder deren Derivat, in einer Sequenz-spezifischen Rekombination von DNA in eukaryorischen Zellen. Die eukaryotische Zelle kann auch im Zellverband eine nicht humanen Organismus, z.B. eines Säugers, vorliegen, der keine Integase oder Xis-Faktor in seinen Zellen enthält. Dieser nicht humane Organismus kann zur Kreuzung mit anderen Organismen verwendet werden, die ihrerseits die Integrase oder den Xis-Faktor in ihren Zellen enthalten, so daß Nachkommen entstehen, in deren Zellen dann die Sequenz-spezifische Rekombination durchgeführt wird. Die Erfindung betrifft somit ebenfalls die Verwendung einer Integrase oder eines Integrase-Gens und eines Xis-Faktors oder eines Xis-Faktor-Gens in einer Sequenz-spezifischen Rekombination in eukaryorischen Zellen.

Wir haben eine Sequenz aus dem menschlichen Genom bestimmt (im folgenden als *att*H bezeichnet), die eine Homologie von etwa 85% zu *att*B aufweist. *Att*H kann als Rekombinationssequenz für die Integration einer fremden DNA in das menschliche Genom verwendet werden. Dazu kann die zweite Rekombinationssequenz *att*P entsprechend modifiziert werden, damit die Integrase die Rekombinationsreaktion mit hoher Effizienz durchführen kann. Wir konnten zeigen, daß *att*H mit einer von uns modifizierten Version von *att*P, die nachfolgend als *att*P* bezeichnet wird und als SEQ ID NO:5 aufgelistet ist, mittels Int-h in *E. coli* rekombiniert werden kann. Experimente mit menschlichen Zellen zeigten, daß *att*H auch als Bestandteil des menschlichen Genoms mit *att*P* rekombiniert wird, wenn Int-h transient von den Zellen synthetisiert wird.

Es ergibt sich daraus die Möglichkeit, eine zirkuläre Fremd-DNA, die eine *att*P-Rekombinationssequenz enthält, stabil und gezielt in den natürlich vorkommenden Lokus *att*H des menschlichen Genoms zu integrieren. Dabei ist *att*H nur ein Beispiel für eine natürliche im menschlichen Genom vorkommende Rekombinationssequenz. Im Rahmen des Human-Genom-Projekts können weitere Sequenzen ermittelt werden, die eine Homologie zu *att*B aufweisen. Diese können ebenfalls für die Integration einer fremden DNA in das menschliche Genom verwendet werden. Abhängig von der im menschlichen Genom vorhandenen zu *att*B homologen Sequenz, wird eine entsprechende *att*P-Rekombinationssequenz in der zirkulären Fremd-DNA gewählt. Bevorzugt ist eine zirkuläre Fremd-DNA, die die Nukleinsäuresequenz der natürlich vorkommenden *att*P-Sequenz enthält. Besonders bevorzugt ist ein Derivat der natürlich vorkommenden *att*P-Sequenz, das maximal sechs, bevorzugt ein bis fünf, insbesondere drei Substitutionen aufweist. Insbesondere bevorzugt ist eine zirkuläre Fremd-DNA, umfassend die *att*P*-Nukleinsäuresequenz gemäß SEQ ID NO:5, die eine Homologie von etwa 95% zu *att*P aufweist.

Dabei kann die Integrase den Zellen entweder als Polypeptid oder über einen Expressionsvektor zugeführt werden. Das Integrase-Gen kann ferner als exprimierbare Nukleinsäuresequenz auf dem DNA-Molekül vorliegen, das die modifizierte oder die natürliche *att*P-Sequenz oder die *att*P*-Sequenz umfaßt.

Die zirkuläre Fremd-DNA, die die natürliche *att*P-Sequenz oder deren Derivat oder Homolog, insbesondere die *att*P*-Sequenz gemäß SEQ ID NO:5 enthält, umfaßt ebenfalls das in das Genom einzuführende therapeutische Gen oder Genfragment. Therapeutische Gene können z.B. das CFTR-Gen, das ADA-Gen, das LDL-Rezeptor-Gen, β-Globin-Gen, FaktorVIII-Gen oder FaktorIX-Gen, alpha-1-Antitrypsin-Gen oder das Dystrophin-Gen sein. Die zirkuläre Fremd-DNA kann z.B. ein bereits in somatischen Gentherapien verwendeter viraler Vektor sein. Der Vektor kann ebenfalls zellspezifisch sein, so daß er nur die für die Gentherapie gewünschten Zellen transfiziert, z.B. die Lungenepithelzellen, die Stammzellen des Knochenmarks, die T-Lymphozyten, die B-Lymphozyten, die Leberzellen, die Nierenzellen, die Nervenzellen, die Skelettmuskelzellen, die hämatopoietischen Stammzellen oder die Fibroblasten. Dem Fachmann ist ersichtlich, daß diese Aufzählung nur eine Auswahl der therapeutischen Gene und Zielzellen darstellt und andere Gene und Zielzellen ebenfalls für eine Gentherapie verwendet werden können. Genfragmente umfassen z.B. Deletionen therapeutischer Gene, einzelne Exons, Antisense-Nukleinsäuresequenzen oder Ribozyme. Genfragmente können ferner Abschnitte eines Gens umfassen, die die Trinukleotidwiederholungen eines Gens, z.B. des Fragilen-X-Gens, enthalten.

Bei Verwendung der Wildtyp-Integrase muß IHF für die Rekombination vorhanden sein. Bevorzugt ist die Verwendung einer modifizierten Integrase, bei der die Rekombination ohne IHF erfolgen kann. Besonders bevorzugt ist die Verwendung von Int-h oder Int-h/218.

Die vorliegende Erfindung betrifft somit die natürlicherweise vorkommende *att*P-Sequenz oder deren Derivat oder deren Homolog. Besonders betrifft die Erfindung die modifizierte *att*P*-Nukleinsäuresequenz gemäß SEQ ID NO:5. Die vorliegenden Erfindung betrifft ferner einen Vektor, umfassend die natürlicherweise vorkommende *att*P-Sequenz oder deren Derivat, insbesondere die *att*P*-Nukleinsäuresequenz gemäß SEQ ID NO:5, und eine weitere Nukleinsäuresequenz, die ein therapeutisches Gen oder dessen Genfragment umfaßt. Bevorzugt ist ein Vektor, bei dem das therapeutische Gen ein CFTR-, ADA-, LDL Rezeptor-, alpha oder beta Globin-, alpha 1-Antitrypsin-, Faktor VIII- oder Faktor IX-Gen oder deren Fragment umfaßt. Der Vektor kann ebenfalls regulatorische DNA-Elemente umfassen, die die Expression des therapeutischen Gens oder dessen Genfragment steuern.

Die vorliegende Erfindung betrifft ferner die Verwendung des Vektors als Arzneimittel in der Human- oder Veterinärmedizin. Ferner betrifft die Erfindung die Verwendung des Vektors zur Herstellung eines Arzneimittels zur somatischen Gentherapie.

Die erfindungsgemäßen Vektoren können z.B. durch intravenöse oder intramuskuläre Injektionen appliziert werden. Die Vektoren können ebenfalls durch Aerosole aufgenommen werden. Weitere Applikationsformen sind für den Fachmann offensichtlich.

### Beispiele

### 1. Herstellung der Expressions- und Substratvektoren

### 1.1 Expressionsvektoren

Die eukaryotischen Expressionsvektoren für Wildtyp-Int (pKEXInt), Int-h (pKEXInt-h), Int-h/218 (pKEXInt-h/218) und pEL13 sind Derivate von pKEX-2-XR (Rittner et al. (1991) Methods Mol. Cell. Biol., **2**, pp. 176). Der Vektor enthält das menschliche Cytomegalovirus-Promoter/Enhancer-Element (CMV) und RNA-splicing und Polyadenylierungs-Signalsequenzen vom kleinen Simian Virus 40 (SV40) Tumor-antigen. Die Int-Gene wurden durch PCR mit den folgenden Primern kloniert: **(3343)** 5'-GCTCTAGACCACCATGGGAAGAAGGCGAAGTCA-3', der am 5'-Ende des Int-Gens liegt und **(3289)** 5'-AAGGAAAGCGGCCGCTCATTATTTGATTTCAATTTTGTCC-3', der am 3'-Ende liegt. Die Amplifikation erfolgte nach einem ersten Denaturierungsschritt bei 95°C (4 min.) mit 30 Zyklen von Denaturierung (95°C, 45 sec.), Primer-Bindung (55°C, 45 sec.), DNA-Synthese (72°C, 2 min.) und einem letzten Syntheseschritt für 4 min bei 72°C. Das resultierende PCR-Fragment wurde in den pKEX-2-XR Vektor mit XbaI and NotI eingesetzt. Int-h wurde vom Vektor pHN16 als Template generiert (Lange-Gustafson, B. und Nash, H. (1989) J. Biol. Chem., **259,** pp. 12724). Wild-typ Int und Int-h/218 wurden mit pTrcInt bzw. pTrcInt-h/218 als Template generiert (Christ, N. und Dröge, P. (1999) J. Mol. Biol., **288,** pp. 825). pEL13 enthält neben dem Int-h Gen zusätzlich eine Kopie von *att*P*.

*Att*P* wurde ausgehend von *att*P durch PCR-Mutagenese konstruiert. Folgende Oligonukleotide wurden dafür eingesetzt:
(**O3**) 5'-GTTCAGCTTTTTGATACTAAGTTG-3',
(**O4**) 5'-CAACTTAGTATCAAAAAGCTGAAC-3',
(**PC**) 5'-TTGATAGCTCTTCCGCTTTCTGTTACAGGTCACTAATACC-3'und
(**PD**) 5'-ACGGTTGCTCTTCCAGCCAGGGAGTGGGACAAAATTGA-3'.

Die Amplifikation erfolgte nach einem ersten Denaturierungsschritt bei 95°C (4 min) mit 30 Zyklen von Denaturierung (95°C, 45 sec.), Primer-Bindung (57°C, 1 min. 30 sec.), DNA-Synthese (72°C, 1 min. 30 sec.) und einem letzten Syntheseschritt für 4 min bei 72°C. Das PCR-Produkt wurde mit dem Restriktionsenzym SapI inkubiert und mit dem SapI-geschnittenen pKEXInt-h ligiert. Das Kontroll-Plasmid pKEX enthält kein Int Gen.

### 1.2 Substratvektoren

Die Substratvektoren sind Derivate von pEGFP (Clontech). Die Rekombinationskassetten stehen unter der Kontrolle des CMV-Promoters, der eine starke, konstitutive Expression garantiert. pGFP*att*B/*att*P wurde konstruiert, indem das GFP-Gen (green flourescence protein) zunächst aus pEGFP mittels AgeI and BamHI ausgeschnitten wurde. Die Wildtyp *att*B-Sequenz wurde als doppelsträngiges Oligonukleotid in den AgeI-geschnittenen Vektor mittels folgender Oligonukleotide eingesetzt:
(**B1OB**) 5'-CCGGTTGAAGCCTGCTTTTTTATACTAACTTGAGCGAACGC-3 und
(**BOB1**) 5'-AATTGCGTTCGCTCAAGTTAGTATAAAAAAGCAGGCTTCAA-3'.

Die Wildtyp *att*P-Sequenz wurde mittels PCR vom Vektor pAB3 (Dröge, P. und Cozzarelli, N. (1989) Proc. Natl. Acad. Sci., **86,** pp. 6062) unter Verwendung folgender Primer amplifiziert:
**(p7)** 5'-TCCCCCCGGGAGGGAGTGGGACAAAATTGA-3'und
**(p6)** 5'-GGGGATCCTCTGTTACAGGTCACTAATAC-3'.

Die Amplifikation erfolgte nach einem ersten Denaturierungsschritt bei 95°C (4 min) mit 30 Zyklen von Denaturierung (95°C, 45 sec.), Primer-Bindung (54°C, 30 sec.), DNA-Synthese (72°C, 30 sec.) und einem letzten Syntheseschritt für 4 min bei 72°C. Das *att*P-enthaltende PCR-Fragment wurde mit XmaI and BamHI geschnitten and mit einem Restriktionsfragment, welches das GFP-Gen enthält, ligiert. Dieses GFP-Restriktionsfragment wurde mit AgeI and EcoRI aus pEGFP hergestellt. Das Ligationsprodukt wurde in den MfeI/BamHI-geschnittenen, *att*Benthaltenden Vektor eingesetzt. Der resultierende Substratvektor enthält das GFP-Gen in invertierter Orientierung in Bezug auf den CMV-Promoter. Dessen Funktionalität in integrativer Rekombination wurde in E.coli mittels Wildtyp-Int getestet.

pGFP*att*L/*att*R ist mit pGFP*att*B/*att*P bis auf die Rekombinationssequenzen identisch. Der Vektor wurde konstruiert, indem zunächst pGFP*att*B/*att*P in *E. coli* rekombiniert wurde, was zur Bildung von *att*L und *att*R führt. Das dann bezüglich des CMV-Promoters korrekt orientierte GFP-Gen wurde durch eine partielle Restriktionsreaktion mit BsiE1 and HindIII ausgeschnitten. Um das GFP-Gen in invertierter Orientierung in Bezug auf den CMV-Promoter einzusetzen, wurde das Gen zunächst mittels PCR unter Verwendung folgender Primer amplifiziert:
(**p2**) 5'-AATCCGCGGTCGGAGCTCGAGATCTGAGTCC-3' und
(**p3**) 5'- AATCCCAAGCTTCCACCATGGTGAGCAAGGG-3' (Fig. 3).

Die Amplifikation erfolgte nach einem ersten Denaturierungsschritt bei 95°C (4 min) mit 30 Zyklen von Denaturierung (95°C, 45 sec.), Primer-Bindung (56°C, 45 sec.), DNA-Synthese (72°C, 1 min.) und einem letzten Syntheseschritt für 4 min bei 72°C. Das PCR-Fragment wurde anschließend mit HindIII and BsiEI geschnitten und in den partiell geschnittenen Vektor integriert. Dieser enthält *att*L and *att*R in invertierter Orientierung. pGFP*att*L/*att*R zeigt demnach dieselbe globale Struktur wie pGFP*att*B/*att*P, mit Ausnahme der Anwesenheit von *att*L/*att*R anstelle von *att*B/*att*P.

Das humane *att*B-Homolog, *att*H, wurde von gereinigter menschlicher DNA mittels PCR unter Verwendung folgender Primer amplifiziert:
(**B3**) 5'-GCTCTAGATTAGCAGAAATTCTTTTTG-3' und
(**B2**) 5'-AACTGCAGTAAAAAGCATGCTCATCACCCC-3'.

Die Amplifikation erfolgte nach einem ersten Denaturierungsschritt bei 95°C (5 min) mit 30 Zyklen von Denaturierung (95°C, 45 sec.), Primer-Bindung (42°C, 1.45 min.), DNA-Synthese (72°C, 1.45 min.) und einem letzten Syntheseschritt für 10 min bei 72°C. Die Primer-Sequenzen zur Generierung von *att*H wurden einer EST entnommen (Accession No.: N31218; EMBL-Database). Die unvollständige Sequenz von *att*H, wie sie in der Datenbank vorliegt, wurde bestätigt und mittels DNA-Sequenzierung des isolierten PCR-Produkts (192 bp) vervollständigt. Das Fragment wurde anschließend mit XbaI and PstI geschnitten und in den entsprechend behandelten Vektor pACYC187 (New England Biolabs) eingesetzt. *att*P* wurde durch PCRgerichtete Mutagenese wie beschrieben hergestellt (Christ, N. und Dröge, P. (1999) J. Mol. Biol., 288, pp.825) und in den *att*H-enthaltenden Vektor in invertierter Orientierung zu *att*H eingesetzt. Diese Konstruktion ergab den Testvektor pACH.

Plasmid-DNAs wurden aus *E. coli* Stamm DH5α (Hanahan, D. (1983) J. Mol. Biol., **166,** pp.557) mittels Affinitätschromatographie (Qiagen, Deutschland) isoliert. Expressions- und Substratvektoren, sowie alle PCR-generierten Konstrukte wurden mittels des fluoreszenzbasierten 373A DNA-Sequenzierungssystems (Applied Biosystems) kontrolliert. PCR-Reaktionen wurden mit dem "Master Mix Kit" (Qiagen, Deutschland) durchgeführt und die Produkte über Agarosegel-Elektrophorese (0.8% w/v) in TBE-Puffer analysiert.

### 2. Zellkultur und Konstruktion der Reporter-Zellinien

Die transienten Expressions- und Rekombinationsanalysen wurden mit einer menschlichen Burkitt's Lymphoma Zellinie (BL60; (Wolf, J. et al., (1990) Cancer Res., **50,** pp. 3095)) durchgeführt. BL60-Zellen wurden in RPMI1640 Medium kultiviert (Life Technologies, Inc.), welches mit 10% fötalem Kälberserum angereichert wurde, und 2 mM L-Glutamin, Streptomyzin (0,1 mg/ml) und Penizillin (100 units/ml) enthält.

BL60-Reporterzellinien, die entweder pGFP*att*B/*att*P oder pGFP*att*L/*att*R stabil ins Genom integriert haben, wurden wie folgt konstruiert: ca. 20 µg von jedem Vektor wurden mit ApaLI linearisiert, durch Phenol/Chloroform-Extraktionen gereinigt, mit Ethanol präzipitiert und in ca. 2 x 10⁷ Zellen mittels Elektroporation bei 260 V und 960 mF unter Verwendung eines "Bio-Rad Gene pulser" eingebracht. Stabile Zellinien wurden mit G418/Genetizin (300 µg/ml) selektioniert und anschließend durch PCR, DNA-Sequenzierung und Southem-Analyse charakterisiert.

### 3. In-Vivo-Rekombinationsanalysen.

Um *in vivo* intramolekulare Rekombination durchzuführen, wurden ca. 2 x 10⁷ Zellen der jeweiligen BL60-Reporterzellinie mit 40 µg von jedem zirkulären Expressionsvektor mittels Elektroporation, wie in Beispiel 2 beschrieben, transfiziert. Nach 72 Stunden wurden die Zellen durch Zentrifugation geerntet und die genomische DNA der Hälfte der Zellen nach Angaben des Herstellers (Qiaamp Blood Kit; Qiagen, Deutschland) über Affinitätschromatographie isoliert. Aus der zweiten Hälfte der Zellen wurde entweder RNA isoliert (Rneasy Kit, Qiagen, Deutschland) oder ein Zelllysat für die Western-Analyse (siehe Beispiel 4) präpariert.

Die Rekombinationsanalysen mit pACH wurden wie bereits zuvor beschrieben (Christ, N. und Dröge, P. (1999) J. Mol. Biol., **288,** pp.825) in *E. coli* durchgeführt. Hierzu wurden die Rekombinasen Int, Int-h und Int-h/218 verwendet. Die erwartete Rekombination von pACH führt zur Inversion und wurde durch Restriktionsanalyse mit HindIII und AvaI nachgewiesen.

Intermolekulare Rekombination zur Integration von pEL13 in den genomisch lokalisierten *att*H von BL60 Zellen wurde wie folgt durchgeführt: 2 x 10⁷ Zellen wurden mit 20 µg zirkulärem pEL13 via Elektroporation, wie oben beschrieben, transfiziert. Nach 48 Stunden wurden die Zellen in einer Konzentration von 1 x 10⁶ Zellen/ml Selektionsmedium (200 µg/ml Hygromyzin B) ausplattiert und für 6-8 Wochen inkubiert. Von einem Teil der jeweiligen überlebenden Zellpopulationen wurde zu diesem Zeitpunkt genomische DNA nach Angaben des Herstellers (Qiaamp Blood Kit; Qiagen, Deutschland) präpariert.

Um intramolekulare, integrative und excisive Rekombination nachzuweisen, wurden 0,4 µg genomische DNA mittels PCR amplifiziert. Hierzu wurden 20-50 pmol der folgenden Primer eingesetzt:
(**p1**) 5'-GGCAAACCGGTTGAAGCCTGCTTTT-3';
(**p2**) 5'-AATCCGCGGTCGGAGCTCGAGATCTGAGTCC-3';
(**p3**) 5'-AATCCCAAGCTTCCACCATGGTGAGCAAGGG-3';
(**p4**) 5'-AACCTCTACAAATGTGGTATGG-3',
(**p5**) 5'-TACCATGGTGATGCGGTTTTG-3';
(**p6**) 5'-GGGGATCCTCTGTTACAGGTCACTAATAC;
(**p7**) 5'-TCCCCCCGGGAGGGAGTGGGACAAAATTGA-3'.

Die Amplifikation erfolgte nach einer ersten Denaturierung bei 95°C (5 min) mit 30 Zyklen von Denaturierung (95°C, 45 sec.), Primerbindung (57°C, 45 sec.), DNA-Synthese (72°C, 1.5 min.) und einem letzten Syntheseschritt für 4 min bei 72°C.

Intermolekulare, integrative Rekombination von pEL13 wurde wie folgt nachgewiesen. Ca. 400 ng der genomischen DNA überlebender Zellpopulationen wurden mit folgenden Oligonukleotiden als Primer für PCR inkubiert:
(**attx1**) 5'-AGTAGGAATTCAGTTGATTCATAGTGACTGC-3' und
(**B2**) 5'-AACTGCAGTAAAAAGCATGCTCATCACCCC-3'.

Die Amplifikation erfolgte nach einer ersten Denaturierung bei 95°C (4 min) mit 30 Zyklen von Denaturierung (95°C, 45 sec.), Primerbindung (52°C, 45 sec.), DNA-Synthese (72°C, 45 sec.) und einem letzten Syntheseschritt für 4 min bei 72°C.

Die Reverse Transkriptase-PCR (RT-PCR) wurde mit 4 µg isolierter RNA durchgeführt. Die cDNAs wurden zunächst nach Angaben des Herstellers unter Verwendung von oligo-dT-Primern synthetisiert (First Strand Synthesis Kit; Pharmacia). Ein Viertel dieser cDNAs wurde als Template für die anschließende PCR eingesetzt unter Verwendung der Primer p3 und p4. Die PCR-Bedingungen waren wie für p1-p7 beschrieben. Um auf Deletion, anstelle von Inversion, zu testen, wurde isolierte genomische DNA mit den Primern p5 und p6 amplifiziert. Beta-actin-Transkripte wurden ausgehend von den cDNAs unter Verwendung der Primer
(**AS**) 5'-TAAAACGCAGCTCAGTAACAGTCCG-3' und
(**S**) 5'-TGGAATCCTGTGGCATCCATGAAAC-3'
analysiert. Die PCR-Bedingungen waren wie für p1-p7 beschrieben.

Southern-Analysen wurden im wesentlichen nach dem Protokoll von Sambrook, J. (1989) Molecular Cloning (2 nd Edt.) Cold Spring Harbor Laboratory Press durchgeführt. Ca. 10 µg genomischer DNA wurde mit NcoI fragmentiert, durch Agarosegel-Elektrophorese (0,8% w/v) in TBE-Puffer aufgetrennt und über Nacht auf eine Nylon-Membran transferiert. Die GFP-Sonde zum Nachweis der Rekombination wurde mittels PCR unter Verwendung der Primer p2 und p3 hergestellt. Die radioaktive Markierung wurde nach den Angaben des Herstellers (Megaprime; Amersham) unter Verwendung von ³²P-markiertem dATP and dCTP (Amersham) durchgeführt.

### 4. Western Analyse

Zelllysate von transient transfizierten Zellen wurden durch Kochen (5 min.) der Zellen in Probenpuffer (New England Biolabs) hergestellt. Die Proteine wurden in einem 12,5%-igen SDS Polyacrylamidgel nach Molekulargewicht aufgetrennt und über Nacht auf eine Nitrocellulose-Membran (Immobilon P, Millipore) transferiert. Die Membran wurde mit 1%-iger Blockierlösung (BM Chemiluminescence Western Blotting Kit; Boehringer Mannheim, Deutschland) behandelt und mit polyklonalen Maus-Antikörpern gegen Wildtyp-Int bei einer Verdünnung von 1:50.000 inkubiert (Antikörper von A. Landy, USA). Die Peroxidasegekoppelten sekundären Antikörper wurden benutzt, um die Position der Integrase im Gel sichtbar zu machen (BM Chemiluminescence Western Blotting Kit; Boehringer Mannheim, Deutschland). *E. coli* Zellextrakte, die Wildtyp-Int enthielten, wurden als Kontrolle verwendet.

### 5. Ergebnisse

### 5.1 Synthese von Int-h in BL60 Zellen

Um zu testen, ob Int-h Rekombination in menschlichen Zellen katalysieren kann, war es zunächst notwendig zu demonstrieren, daß die Rekombinase von den Zellen synthetisiert werden kann. Hierfür wurde der eukaryotische Expressionsvektor, pKEXInt-h, der das Int-h Gen unter der Kontrolle des CMV-Promoters enthält, eingesetzt. Nach dem Einbringen von pKEXInt-h in zwei verschiedene BL60-Reporterzellinien, B2 and B3, konnten vollständige und korrekt modifizierte Transkripte, spezifisch für das Int-h Gen, durch RT-PCR-Analyse nachgewiesen werden. Zelllysate wurden 72 Stunden nach Elektroporation mit pKEXInt-h durch eine Westem-Analyse untersucht. Der Nachweis der Rekombinase erfolgte mittels polyklonalen Antikörpern der Maus, die gegen Wildtyp-Int gerichtet sind. Als Kontrolle wurde pKEX in die Zellen eingebracht.

Die Ergebnisse zeigten, daß ein Protein mit dem erwarteten Molekulargewicht in den Zellen vorhanden war, die mit pKEXInt-h in der Elektroporation behandelt wurden. Dieses Protein war nicht nachweisbar, wenn der Kontrollvektor pKEX eingesetzt wurde.

### 5.2 Int-h katalysierte integrative, intramolekulare Rekombination in menschlichen Zellen

Die Westem-Analyse zeigte, daß, ausgehend vom Vektor pKEXInt-h, das Int-h Protein von den zwei Reporterzellinien synthetisiert wird. Diese Zellen enthalten einen Substratvektor, pGFP*att*B/*att*P, als stabil in ihr Genom integrierte Fremd-DNA. Die zwei Rekombinationssequenzen für integrative Rekombination, *att*B und *att*P, befinden sich in invertierter Orientierung zueinander und flankieren das Gen für GFP. Das GFP-Gen selbst befindet sich in invertierter Orientierung zum CMV-Promoter, der stromaufwärts von *att*B liegt. Rekombination zwischen *att*B and *att*P durch Int-h führte zur Inversion des GFP-Gens und somit zu dessen Expression. Es wurden insgesamt drei Reporterzellinien (B1-B3) konstruiert. Southern-Analyse ihrer genomischen DNA zeigte, daß B1 und B3 mehrere Kopien von pGFP*att*B/*att*P als direkte Wiederholungen im Genom integriert haben. Die Zellinie B2 enthält dagegen nur eine Kopie. Die integrierten Sequenzen wurden durch PCR und anschließende Sequenzierung verifiziert.

Um die Rekombination zwischen *att*B and *att*P zu testen, wurden pKEXInt-h und pKEX separat in diese Zellinien eingebracht. 72 Stunden nach Elektroporation wurden die Zellen geerntet, RNA von einem Teil der Zellen isoliert und auf GFP-Expression mittels RT-PCR unter Verwendung des Primerpaars p3/p4 untersucht. Diese Primer amplifizierten ein 0,99kb langes DNA-Fragment nur dann, wenn das GFP-Gen infolge der Rekombination invertiert wurde. Die Ergebnisse zeigten, daß das Produkt in allen drei Zellinien nachzuweisen war. Wenn pKEX in die Zellen eingebracht wurde, konnte kein Produkt nachgewiesen werden. DNA-Sequenzanalysen der isolierten PCR-Produkte bestätigten, daß die kodierende Region des GFP-Gens transkribiert wurde und das *att*R, anstelle von *att*P, im Transkript nachzuweisen war. Als Kontrolle für den Gehalt an RNA in allen sechs Zellpräparationen sowie für erfolgreiche Erst-Strang DNA-Synthese durch die Reverse Transkriptase, wurde das endogene β-Aktin-Transkript durch PCR analysiert. Die Resultate zeigten, daß das Transkript in nahezu gleichen Mengen vorlag.

Rekombination wurde auch durch direkte PCR von genomischer DNA nachgewiesen. Die Ergebnisse zeigten, daß die erwarteten Produkte durch die Primerpaare p3/p4 (0,99 kb) und p1/p2 (0,92 kb) nur dann nachzuweisen waren, wenn pKEXInt-h in die Zellen eingebracht wurde. Die Analyse dieser Produkte durch DNA-Sequenzierung bestätigte, daß *att*R und *att*L im Genom anwesend waren und daß das GFP-Gen durch die Rekombination invertiert wurde. Diese Experimente wurden insgesamt drei Mal wiederholt, wobei Rekombination zwischen *att*B und *att*P durch RT-PCR und/oder PCR in allen drei Zellinien nachzuweisen war. Ein Nachweis der Deletion des GFP-Gens durch PCR mit Primerpaar p5/p6 verlief negativ. Nur das erwartete 1,3 kb Fragment, daß vom integrierten Vektor resultierte, konnte amplifiziert werden.

In einem weiteren Experiment wurde das stärkste Signal, daß Inversion zwischen *att*B und *att*P in der PCR anzeigte, wiederholt mit der Zellinie B3 erhalten. Genomische DNA wurde daraufhin durch NcoI fragmentiert und über eine Southern-Analyse untersucht. Hierfür wurde das GFP-Gen als Sonde eingesetzt. Die Ergebnisse zeigten, daß das durch die Inversion zwischen *att*B and *att*P erwartete Restriktionsfragment genomischer DNA in der Zellinie B3 nachzuweisen war.

Um zu testen, ob Wildtyp-Int und die Mutante Int-h/218 ebenfalls intramolekulare, integrative Rekombination katalysieren konnten, wurden in einem weiteren Experiment die Vektoren pKEXInt-h, pKEXInt-h/218, pKEXInt und als Kontrolle pKEX durch Elektroporation, wie in Beispiel 2 beschrieben, in die Reporterzellinie B3 eingebracht. Nach 72 Stunden wurde genomische DNA isoliert und über PCR mit den Primerpaaren p5/p7 und p3/p4, wie in Beispiel 3 beschrieben, auf Rekombination getestet. Die Ergebnisse zeigten, daß beide Int-Mutanten Rekombination zwischen *att*B und *att*P katalysieren konnten, Wildtyp-Int jedoch inaktiv war.

### 5.3 Excisive Rekombination zwischen attL und attR war nicht nachzuweisen

Da Int-h auch excisive Rekombination zwischen *att*L and *att*R in der Abwesenheit der Kofaktoren IHF und Xis katalysieren konnte, wurden drei BL60-Reporterzellinien konstruiert, die den Vektor pGFP*att*L/*att*R stabil im Genom integriert haben. Diese Zellinien enthalten das GFP Gen wiederum in invertierter Orientierung in Bezug auf den CMV-Promoter, allerdings flankiert von *att*L und *att*R anstelle von *att*B und *att*P. Die Rekombinationsanalysen wurden, wie in Beispiel 3 beschrieben, mit pKEXInt-h als Expressionsvektor für die Rekombinase durchgeführt, zeigten jedoch, daß weder Inversion noch Deletion zwischen *att*L und *att*R mittels RT-PCR oder PCR nachzuweisen war.

### 5.4 Identifikation und Charakterisierung einer natürlich vorkommenden, attB-ähnlichen Nukleotidsequenz im menschlichen Genom

Wie in Beispiel 3 gezeigt, katalysierten beide Mutanten der Int-Rekombinase integrative, intramolekulare Rekombination in menschlichen Zellen. Eine der beiden an dieser Reaktion beteiligten Rekombinationssequenzen, *att*B, ist 21 Basenpaare (bp) lang und natürlicher Bestandteil des *E. coli* Genoms. Es konnte gezeigt werden, daß Int-h einige Abweichungen in der Sequenz der sog. "core" Erkennungsregion von *att*B für eine Rekombination mit *att*P toleriert (Nash (1981) Annu. Rev. Genet., **15**, pp143). Aus statistischen Überlegungen ist das Vorhandensein einer funktionellen, *att*B homologen Sequenz im menschlichen Genom daher möglich. In einer Datenbanksuche konnten wir eine noch unvollständige Sequenz als Teil eines "expressed sequenz tag" (EST) identifizieren. Diese Sequenz wurde anschließend durch PCR aus menschlicher DNA isoliert und kloniert. DNA-Sequenzanalyse vervollständigte die Sequenz und eine weitergehende Southem-Analyse genomischer DNA der BL60-Zellen zeigte, daß diese Sequenz Teil eines noch unbekannten menschlichen Gens ist, das in einer Kopie im Genom vorkommt.

Diese Sequenz, die im folgenden als *att*H bezeichnet wird, weicht von der Wildtyp *att*B-Sequenz an drei Positionen ab. Zwei der Nukleotide befinden sich in der linken (B) "core"-Int-Erkennungsregion und eines ist Teil der sog. "overlap"-Region. Da die Identität der "overlap"-Region zwischen zwei Rekombinationssequenzen eine Voraussetzung für eine effiziente Rekombination durch Int-h ist, wurde das entsprechende Nukleotid an Position 0 im "overlap" von *att*P von Thymidin in Guanin verändert, was zu *att*P* führte. *att*H und *att*P* wurden als invertierte Sequenzen in einen Vektor eingesetzt (pACH) und auf Rekombination in *E. coli* getestet. Die Ergebnisse zeigten, daß Int-h und Int-h/218 Inversion zwischen *att*H and *att*P* in der Anwesenheit von IHF katalysierten. DNA-Sequenz-Analysen der isolierten Rekombinationsprodukte bestätigten, daß Rekombination zwischen *att*H und *att*P* nach dem erwarteten Mechanismus abgelaufen war. Wildtyp-Int kann dagegen *att*H/*att*P* auch in Anwesenheit von IHF nur sehr ineffizient rekombinieren. *att*H ist demnach eine potentielle Integrationssequenz für Int-h-katalysierte Integration fremder DNA, die eine Kopie von *att*P* enthält.

### 5.5 Integrative, intermolekulare Rekombination zwischen attH und attP* in menschlichen Zellen

Um zu testen, ob *att*H als natürlicher Bestandteil des menschlichen Genoms mit *att*P* in einer intermolekularen Reaktion rekombinieren kann, wurde pEL13 konstruiert. Dieser Vektor enthielt, neben dem Int-h-Gen unter der Kontrolle des CMV-Promoters, eine Kopie von *att*P* und, als Selektionsmarker, das Resistenzgen Hygromyzin. Int-h konnte nach Einbringen von pEL13 in BL60-Zellen synthetisiert werden und katalysierte die intermolekulare Rekombination zwischen *att*P* als Bestandteil von pEL13 und genomischem *att*H.

pEL13 wurde durch Elektroporation, wie in Beispiel 2 beschrieben, in BL60-Zellen eingebracht und diese nach 72 Stunden unter Selektionsdruck gebracht und verdünnt. Überlebende Zellpopulationen wurden nach 6-8 Wochen durch PCR mit dem Primerpaar attx1/B2 auf Rekombinationsereignisse untersucht. Die Ergebnisse zeigten, daß in 13 der überlebenden 31 Zellpopulationen eine Integration in *att*H nachzuweisen war. DNA-Sequenzanalysen der aus verschiedenen Ansätzen isolierten PCR-Produkte bestätigten deren Identität als Rekombinationsprodukte.

## Patentansprüche

1. Verfahren zur Sequenz-spezifischen Rekombination von DNA in einer eukaryotischen Zelle *in vitro,* umfassend
a) das Einführen in eine Zelle einer ersten DNA-Sequenz, umfassend:
eine *att*B-Sequenz gemäß SEQ ID NO:1 oder deren Derivat, oder
eine *att*P-Sequenz gemäß SEQ ID NO:2 oder deren Derivat, oder
eine *att*L-Sequenz gemäß SEQ ID NO:3 oder deren Derivat, oder
eine *att*R-Sequenz gemäß SEQ ID NO:4 oder deren Derivat,
b) das Einführen in die Zelle einer zweiten DNA-Sequenz, umfassend:
eine *att*P-Sequenz gemäß SEQ ID NO:2 oder deren Derivat, falls die erste DNA-Sequenz eine *att*B-Sequenz gemäß SEQ ID NO:1 oder deren Derivat umfasst;
oder
eine *att*B-Sequenz gemäß SEQ ID NO:1 oder deren Derivat, falls die erste DNA-Sequenz eine *att*P-Sequenz gemäß SEQ ID NO:2 oder deren Derivat umfasst;
oder
eine *att*L-Sequenz gemäß SEQ ID NO:3 oder deren Derivat, falls die erste DNA-Sequenz eine *att*R-Sequenz gemäß SEQ ID NO:4 oder deren Derivat umfasst;
oder
eine *att*R-Sequenz gemäß SEQ ID NO:4 oder deren Derivat, falls die erste DNA-Sequenz eine *att*L-Sequenz gemäß SEQ ID NO:3 oder deren Derivat umfasst;
c) das Durchführen der Sequenz-spezifischen Rekombination durch Einwirken einer Bakteriophagen *Lambda*-Integrase Int,
wobei der Ausdruck "Derivat" *att*B-, *att*P-, *att*L- und *att*R-Sequenzen bezeichnet, die gegenüber den natürlicherweise vorkommenden Rekombinationssequenzen Modifikationen in Form von einer oder mehreren, aber maximal sechs Substitutionen aufweisen.

2. Verfahren zur Sequenz-spezifischen Rekombination von DNA in einer eukaryotischen Zelle *in vitro,* die in ihrem Genom die erste DNA-Sequenz enthält, entweder natürlicherweise in der Zelle vorliegend oder mit Hilfe von DNA-Rekombination zuvor eingebracht, umfassend die Schritte b) und c), wie sie in Anspruch 1 definiert wurden.

3. Verfahren nach Anspruch 1 oder 2, wobei die erste DNA-Sequenz eine *att*B-Sequenz gemäß SEQ ID NO:1 oder deren Derivat umfaßt, und die zweite DNA-Sequenz eine *att*P-Sequenz gemäß SEQ ID NO:2 oder deren Derivat umfaßt.

4. Verfahren nach Anspruch 1 oder 2, wobei die erste DNA-Sequenz eine *att*L-Sequenz gemäß SEQ ID NO:3 oder deren Derivat umfaßt, und die zweite DNA-Sequenz eine *att*R-Sequenz gemäß SEQ ID NO:4 oder deren Derivat umfaßt, wobei in Schritt c) zusätzlich ein Xis-Faktor vorhanden ist.

5. Verfahren nach einem der Ansprüche 1 bis 4, wobei zusätzlich eine dritte oder eine dritte und vierte DNA-Sequenz, umfassend ein Int-Gen oder ein Int-Gen und ein Xis-Faktor-Gen, in die Zelle eingeführt wird/werden.

6. Verfahren nach Anspruch 5, wobei die dritte oder die dritte und/oder vierte DNA-Sequenz ferner eine regulatorische DNA-Sequenz umfaßt/umfassen, die eine räumliche und/oder zeitliche Expression des Int-Gens und/oder des Xis-Faktor-Gens bewirkt/bewirken.

7. Verfahren nach einem der Ansprüche 1 bis 6, wobei Int eine modifizierte Integrase ist.

8. Verfahren nach Anspruch 7, wobei die modifizierte Int Int-h oder Int-h/218 ist.

9. Verfahren nach einem der Ansprüche 1 bis 8, wobei in Schritt c) zusätzlich ein "Integration Host Factor" (IHF) beteiligt ist.

10. Verfahren nach einem der Ansprüche 1 bis 9, wobei die erste und/oder zweite DNA-Sequenz ferner DNA-Sequenzen umfaßt/umfassen, die eine Integration der ersten und/oder zweiten DNA-Sequenz in das Genom der eukaryotischen Zellen mittels homologer Rekombination bewirkt/bewirken.

11. Verfahren nach einem der Ansprüche 1 bis 10, wobei die erste und/oder zweite DNA-Sequenz ferner eine Nukleinsäuresequenz umfaßt/umfassen, die ein Polypeptid von Interesse codiert.

12. Verfahren nach Anspruch 11, wobei das Polypeptid von Interesse ein Strukturprotein, ein endogenes oder exogenes Enzym, ein regulatorisches Protein oder ein Markerprotein ist.

13. Verfahren nach einem der Ansprüche 1 und 3 bis 12, wobei die erste und zweite DNA-Sequenz auf demselben DNA-Molekül in die eukaryotische Zelle eingeführt werden.

14. Verfahren nach einem der Ansprüche 1 bis 13, wobei die eukaryotische Zelle eine Säugerzelle ist.

15. Verfahren nach Anspruch 14, wobei die Säugerzelle eine menschliche Zelle, eine Affen-, Maus-, Ratten-, Kaninchen-, Hamster-, Ziege-, Kuh-, Schaf- oder Schweinezelle ist.

16. Verfahren nach einem der Ansprüche 1 bis 3 und 5 bis 15, ferner umfassend
d) das Durchführen einer zweiten Sequenz-spezifischen Rekombination von DNA, nach einer ersten Sequenz-spezifischen Rekombination nach den Schritten a) bis c), wobei die erste DNA-Sequenz *att*B gemäß SEQ ID NO:1 oder deren Derivat und zweite die DNA-Sequenz *att*P gemäß SEQ ID NO:2 oder deren Derivat umfaßt oder umgekehrt, durch Einwirken einer Int und eines Xis-Faktors.

17. Verfahren nach Anspruch 16, wobei ferner eine weitere DNA-Sequenz, umfassend ein Xis-Faktor-Gen, in die Zellen eingeführt wird.

18. Verfahren nach Anspruch 17, wobei die weitere DNA-Sequenz ferner eine regulatorische DNA-Sequenz umfaßt, die eine räumliche und/oder zeitliche Expression des Xis-Faktor-Gens bewirkt.

19. Verwendung einer *att*B-Sequenz gemäß SEQ ID NO:1 oder deren Derivat und einer *att*P-Sequenz gemäß SEQ ID NO:2 oder deren Derivat, oder einer *att*L-Sequenz gemäß SEQ ID NO:3 oder deren Derivat und einer *att*R-Sequenz gemäß SEQ ID NO:4 oder deren Derivat, in einer Sequenz-spezifischen Rekombination von DNA in eukaryotischen Zellen *in vitro*, wobei der Ausdruck "Derivat" *att*B-, *att*P-, *att*L- und *att*R-Sequenzen bezeichnet, die gegenüber den natürlicherweise vorkommenden Rekombinationssequenzen Modifikationen in Form von einer oder mehreren, aber maximal sechs Substitutionen aufweisen.

20. Modifizierte *att*P Nukleinsäure gemäß SEQ ID NO:5 oder deren Derivat, wobei der Ausdruck "Derivat" *att*B-, *att*P-, *att*L- und *att*R-Sequenzen bezeichnet, die gegenüber SEQ ID NO:5 Modifikationen in Form von einer oder mehreren, aber maximal sechs Substitutionen aufweisen, mit der Maßgabe, daß das Derivat nicht der Wildtyp *att*P-Sequenz entspricht.

21. Vektor, umfassend die Nukleinsäuresequenz nach Anspruch 20 und eine weitere Nukleinsäuresequenz, die ein therapeutisches Gen oder dessen DNA-Fragment codiert.

22. Vektor nach Anspruch 21, wobei das therapeutische Gen das CFTR-Gen, ADA-Gen, LDL-Rezeptor-Gen, β-Globin-Gen, FaktorVIII-Gen oder FaktorIX-Gen, alpha-1-Antitrypsin-Gen oder das Dystrophin-Gen oder ein Genfragment eines dieser Gene ist.

23. Vektor nach Anspruch 21 oder 22, wobei die weitere Nukleinsäuresequenz ferner Expressions- und/oder Transkriptionselemente umfaßt.

24. Vektor nach einem der Ansprüche 21 bis 23 zur Verwendung als Arzneimittel in der Human- oder Veterinärmedizin.

25. Verwendung des Vektors nach einem der Ansprüche 21 bis 23 zur Herstellung eines Arzneimittels zur somatischen Gentherapie.

26. Eukaryotische Zelle, enthaltend *att*B- und *att*P- oder *att*L- und *att*R-Sequenzen, oder Derivate davon, erhältlich **dadurch**, daß die in Anspruch 1 und 2 genannte eukaryotische Zelle dem Verfahren nach einem der Ansprüche 1 bis 18 unterworfen wurde.

27. Transgener, nicht humaner Organismus, enthaltend mindestens eine Zelle nach Anspruch 26.

28. Organismus nach Anspruch 27, wobei dieser Organismus eine Maus, eine Ratte, ein Kaninchen oder ein Hamster ist.

## Claims

1. Method of sequence specific recombination of DNA in a eukaryotic cell *in vitro,* comprising
a) introducing into a cell a first DNA sequence, comprising:
an *att*B sequence according to SEQ ID NO:1 or a derivative thereof, or
an *att*P sequence according to SEQ ID NO:2 or a derivative thereof, or
an *att*L sequence according to SEQ ID NO:3 or a derivative thereof, or
an *att*R sequence according to SED ID NO:4 or a derivative thereof,
b) introducing into the cell a second DNA sequence, comprising:
an *att*P sequence according to SEQ ID NO:2 or a derivative thereof, if the first DNA sequence comprises an *att*B sequence according to SEQ ID NO:1 or a derivative thereof;
or
an *att*B sequence according to SEQ ID NO:1 or a derivative thereof, if the first DNA sequence comprises an *att*P sequence according to SEQ ID NO:2 or a derivative thereof;
or
an *att*L sequence according to SEQ ID NO:3 or a derivative thereof, if the first DNA sequence comprises an *att*R sequence according to SEQ ID NO:4 or a derivative thereof;
or
an *att*R sequence according to SEQ ID NO:4 or a derivative thereof, if the first DNA sequence comprises an *att*L sequence according to SEQ ID NO:3 or a derivative thereof;
c) performing the sequence specific recombination effectuated by a bacteriophage *lambda* integrase Int, wherein the expression "derivative" refers to *att*B, *att*P, *att*L and *att*R sequences exhibiting modifications in the form of one or more, but at most six substitutions in contrast to naturally occurring recombination sequences.

2. Method of sequence specific recombination of DNA in a eukaryotic cell *in vitro* comprising in its genome the first DNA sequence, either naturally occurring in the cell or introduced before by means of DNA recombination, comprising steps b) and c) as defined in claim 1.

3. Method according to claim 1 or 2, wherein the first DNA sequence comprises an *att*B sequence according to SEQ ID NO: 1 or a derivative thereof, and the second DNA sequence comprises an *att*P sequence according to SEQ ID NO:2 or a derivative thereof.

4. Method according to claim 1 or 2, wherein the first DNA sequence comprises an *att*L sequence according to SEQ ID NO:3 or a derivative thereof and the second DNA sequence comprises an *att*R sequence according to SEQ ID NO:4 or a derivative thereof, wherein in step c) additionally a Xis factor is present.

5. Method according to anyone of claims 1 to 4, wherein additionally a third or a third and a fourth DNA sequence, comprising an Int gene or an Int gene and a Xis factor gene, is/are introduced into the cell.

6. Method according to claim 5, wherein the third and the third and/or fourth DNA sequence further comprises/comprise a regulatory DNA sequence effecting a spatial and/or temporal expression of the Int gene and/or of the Xis factor gene.

7. Method according to anyone of claims 1 to 6, wherein Int is a modified integrase.

8. Method according to claim 7, wherein the modified Int is Int-h or Int-h/218.

9. Method according to anyone of claims 1 to 8, wherein in step c) additionally an "integration host factor" (IHF) is involved.

10. Method according to anyone of claims 1 to 9, wherein the first and/or second DNA sequence further comprises/comprise DNA sequences effecting an integration of the first and/or second DNA sequence into the genome of the eukaryotic cells by means of homologous recombination.

11. Method according to anyone of claims 1 to 10, wherein the first and/or second DNA sequence further comprises/comprise a nucleic acid sequence encoding a polypeptide of interest.

12. Method according to claim 11, wherein the polypeptide of interest is a structural protein, an endogenous or exogenous enzyme, a regulatory protein or a marker protein.

13. Method according to anyone of claims 1 and 3 to 12, wherein the first and second DNA sequence are introduced into the eukaryotic cell on the same DNA molecule.

14. Method according to anyone of claims 1 to 13, wherein the eukaryotic cell is a mammalian cell.

15. Method according to claim 14, wherein the mammalian cell is a human cell, a simian cell, a murine cell, a rat cell, a rabbit cell, a hamster cell, a goat cell, a bovine cell, a sheep cell or a porcine cell.

16. Method according to anyone of claims 1 to 3 and 5 to 15, further comprising
d) performing a second sequence specific recombination of DNA after a first sequence specific recombination after steps a) to c), wherein the first DNA sequence comprises *att*B according to SEQ ID NO: or a derivate thereof and the second comprises the DNA sequence *att*P according to SEQ ID NO:2 or a derivative thereof or *vice versa*, by the action of an Int and a Xis factor.

17. Method according to claim 16, wherein further a further DNA sequence comprising a Xis factor gene is introduced into the cells.

18. Method according to claim 17, wherein the further DNA sequence further comprises a regulatory DNA sequence effecting a spatial and/or temporal expression of the Xis factor gene.

19. Use of an *att*B sequence according to SEQ ID NO: 1 or a derivative thereof and of an *att*P sequence according to SEQ ID NO:2 or a derivative thereof, or of an *att*L sequence according to SEQ ID NO:3 or a derivative thereof and of an *att*R sequence according to SEQ ID NO:4 or a derivative thereof in a sequence specific recombination of DNA in eukaryotic cells *in vitro,* wherein the expression "derivative" refers to *att*B, *att*P, *att*L and *att*R sequences exhibiting modifications in the form of one or more, but at most six substitutions in contrast to naturally occurring recombination sequences.

20. Modified *att*P nucleic acid according to SEQ ID NO:5 or a derivative thereof, wherein the expression "derivative" refers to *att*B, *att*P, *att*L and *att*R sequences exhibiting modifications in the form of one or more, but at most six substitutions in contrast to SEQ ID NO:5, provided that the derivative does not correspond to the wild type *att*P sequence.

21. Vector comprising the nucleic acid sequence according the claim 20 and a further nucleic acid sequence encoding a therapeutic gene or a DNA fragment thereof.

22. Vector according to claim 21, wherein the therapeutic gene is the CFTR gene, the ADA gene, the LDL receptor gene, the β-globin gene, the factor VIII gene or the factor IX gene, the alpha-1-antitrypsin gene or the dystrophin gene or a gene fragment of anyone of said genes.

23. Vector according to claim 21 or 22, wherein the further nucleic acid sequence further comprises expression and/or transcription elements.

24. Vector according to anyone of the claims 21 to 23 for the use as a medicament in human or veterinary medicine.

25. Use of the vector according to anyone of claims 21 to 23 for the manufacture of a medicament for somatic gene therapy.

26. Eukaryotic cell comprising *att*B and *att*P or *att*L and *att*R sequences or derivatives thereof, obtainable by subjecting the eukaryotic cell mentioned in claim 1 and 2 to the method according to anyone of claims 1 to 18.

27. Transgenic, non-human organism comprising at least one cell according to claim 26.

28. Organism according to claim 27, wherein said organism is a mouse, a rat, a rabbit or a hamster.

## Revendications

1. Procédé pour la recombinaison séquence-spécifique d'ADN dans une cellule eucaryote in vitro, comprenant :
a) l'introduction dans une cellule d'une première séquence d'ADN comprenant :
une séquence attB selon SEQ ID n° 1 ou un dérivé de celle-ci, ou
une séquence attP selon SEQ ID n° 2 ou un dérivé de celle-ci, ou
une séquence attL selon SEQ ID n° 3 ou un dérivé de celle-ci, ou
une séquence attR selon SEQ ID n° 4 ou un dérivé de celle-ci,
b) l'introduction dans la cellule d'une seconde séquence d'ADN comprenant :
une séquence attP selon SEQ ID n° 2 ou un dérivé de celle-ci, dans le cas où la première séquence d'ADN comprend une séquence attB selon SEQ ID n° 1 ou un dérivé de celle-ci; ou
une séquence attB selon SEQ ID n° 1 ou un dérivé de celle-ci, dans le cas où la première séquence d'ADN comprend une séquence attP selon SEQ ID n° 2 ou un dérivé de celle-ci ; ou
une séquence attL selon SEQ ID n° 3 ou un dérivé de celle-ci, dans le cas où la première séquence d'ADN comprend une séquence attR selon SEQ ID n° 4 ou un dérivé de celle-ci; ou
une séquence attR selon SEQ ID n° 4 ou un dérivé de celle-ci, dans le cas où la première séquence d'ADN comprend une séquence attL selon SEQ ID n° 3 ou un dérivé de celle-ci ;
c) la réalisation de la recombinaison séquence-spécifique par l'action de l'intégrase Int d'un bactériophage lambda,
l'expression « dérivé » désignant les séquences attB, attP, attL et attR qui présentent, par rapport aux séquences de recombinaison naturelles, des modifications sous la forme d'une ou de plusieurs, mais n'excédant pas six, substitutions.

2. Procédé pour la recombinaison séquence-spécifique d'ADN in vitro dans une cellule eucaryote qui contient dans son génome la première séquence d'ADN, qu'elle soit présente naturellement dans la cellule ou qu'elle ait été préalablement introduite à l'aide de la recombinaison d'ADN, comprenant les étapes b) et c) telles qu'elles ont été définies dans la revendication 1.

3. Procédé selon la revendication 1 ou 2, dans lequel la première séquence d'ADN comprend une séquence attB selon SEQ ID n° 1 ou un dérivé de celle-ci et la seconde séquence d'ADN comprend une séquence attP selon SEQ ID n° 2 ou un dérivé de celle-ci.

4. Procédé selon la revendication 1 ou 2, dans lequel la première séquence d'ADN comprend une séquence attL selon SEQ ID n° 3 ou un dérivé de celle-ci et la seconde séquence d'ADN comprend une séquence attR selon SEQ ID n° 4 ou un dérivé de celle-ci, un facteur Xis étant de plus présent à l'étape c).

5. Procédé selon l'une des revendications 1 à 4, dans lequel on introduit en outre dans la cellule une troisième séquence ou des troisième et quatrième séquences d'ADN comprenant un gène Int ou un gène Int et un gène du facteur Xis.

6. Procédé selon la revendication 5, dans lequel la troisième séquence ou la troisième et/ou quatrième séquence(s) d'ADN comprend/comprennent en outre une séquence d'ADN régulatrice qui effectue une expression spatiale et/ou temporelle du gène Int et/ou du gène du facteur Xis.

7. Procédé selon l'une des revendications 1 à 6, dans lequel Int est une intégrase modifiée.

8. Procédé selon la revendication 7, dans lequel l'Int modifiée est Int-h ou Int-h/218.

9. Procédé selon l'une des revendications 1 à 8, dans lequel, à l'étape c), un « facteur d'intégration de l'hôte » (IHF) est de plus impliqué.

10. Procédé selon l'une des revendications 1 à 9, dans lequel la première et/ou seconde séquence(s) d'ADN comprend/comprennent en outre des séquences d'ADN qui réalisent une intégration de la première et/ou seconde séquence(s) d'ADN dans le génome des cellules eucaryotes au moyen d'une recombinaison homologue.

11. Procédé selon l'une des revendications 1 à 10, dans lequel la première et/ou seconde séquence(s) d'ADN comprend/comprennent en outre une séquence d'acides nucléiques qui code pour un polypeptide d'intérêt.

12. Procédé selon la revendication 11, dans lequel le polypeptide d'intérêt est une protéine de structure, une enzyme endogène ou exogène, une protéine régulatrice ou une protéine marqueur.

13. Procédé selon l'une des revendications 1 et 3 à 12, dans lequel la première et la seconde séquences d'ADN sont introduites dans la cellule eucaryote sur la même molécule d'ADN.

14. Procédé selon l'une des revendications 1 à 13, dans lequel la cellule eucaryote est une cellule de mammifère.

15. Procédé selon la revendication 14, dans lequel la cellule de mammifère est une cellule humaine, une cellule de singe, de souris, de rat, de lapin, de hamster, de chèvre, de vache, de mouton ou de porc.

16. Procédé selon l'une des revendications 1 à 3 et 5 à 15, comprenant en outre :
d) la réalisation d'une seconde recombinaison séquence-spécifique d'ADN, après une première recombinaison séquence-spécifique selon les étapes a) à c), comprenant de cette façon la première séquence d'ADN attB selon SEQ ID n° 1 ou un dérivé de celle-ci et la seconde séquence d'ADN attP selon SEQ ID n° 2 ou un dérivé de celle-ci, ou inversement, par l'action d'une Int et d'un facteur Xis.

17. Procédé selon la revendication 16, dans lequel on introduit en outre dans les cellules une autre séquence d'ADN comprenant un gène du facteur Xis.

18. Procédé selon la revendication 17, dans lequel l'autre séquence d'ADN comprend en outre une séquence d'ADN régulatrice qui effectue une expression spatiale et/ou temporelle du gène du facteur Xis.

19. Utilisation d'une séquence attB selon SEQ ID n° 1 ou d'un dérivé de celle-ci et d'une séquence attP selon SEQ ID n° 2 ou d'un dérivé de celle-ci, ou d'une séquence attL selon SEQ ID n° 3 ou d'un dérivé de celle-ci et d'une séquence attR selon SEQ ID n° 4 ou d'un dérivé de celle-ci, dans une recombinaison séquence-spécifique d'ADN dans des cellules eucaryotes in vitro, dans laquelle l'expression « dérivé » désigne les séquences attB, attP, attL et attR qui présentent, par rapport aux séquences de recombinaison naturelles, des modifications sous la forme d'une ou de plusieurs, mais n'excédant pas six, substitution(s).

20. Acide nucléique attP modifié selon SEQ ID n° 5 ou un dérivé de celle-ci, dans lequel l'expression « dérivé » désigne les séquences attB, attP, attL et attR qui présentent, par rapport à SEQ ID n° 5 des modifications sous la forme d'une ou de plusieurs, mais n'excédant pas six, substitution(s), à condition que le dérivé ne corresponde pas à la séquence attP de type sauvage.

21. Vecteur comprenant la séquence d'acides nucléiques selon la revendication 20 et une autre séquence d'acides nucléiques qui code pour un gène thérapeutique ou son fragment d'ADN.

22. Vecteur selon la revendication 21, dans lequel le gène thérapeutique est le gène CFTR, le gène de l'ADA, le gène du récepteur des LDL, le gène de la β-globine, le gène du facteur VIII ou le gène du facteur IX, le gène de l'alpha-1 antitrypsine ou le gène de la dystrophine ou un fragment génique de l'un de ces gènes.

23. Vecteur selon la revendication 21 ou 22, dans lequel l'autre séquence d'acides nucléiques comprend en outre des éléments d'expression et/ou de transcription.

24. Vecteur selon l'une des revendications 21 à 23 destiné à être utilisé comme médicament en médecine humaine ou vétérinaire.

25. Utilisation du vecteur selon l'une des revendications 21 à 23 pour la fabrication d'un médicament destiné à la thérapie génique somatique.

26. Cellule eucaryote contenant les séquences attB et attP ou attL et attR ou des dérivés de celles-ci, disponible en ce que la cellule eucaryote mentionnée dans les revendications 1 et 2 a été soumise au procédé selon l'une des revendications 1 à 18.

27. Organisme non humain transgénique contenant au moins une cellule selon la revendication 26.

28. Organisme selon la revendication 27, dans lequel cet organisme est une souris, un rat, un lapin ou un hamster.
